# EUROPEAN PATENT APPLICATION

(11) **EP 1 239 050 A1**
(43) Date of publication of application: **11.09.2002**
(21) Application number: 00981649.7
(22) Date of filing: 08.12.2000
(51) Int. Cl.: C12Q 1/68, C12Q 1/02, C12N 15/12, G01N 33/50, G01N 33/563, C07K 16/18, G01N 33/92, A61K 48/00, A61P 3/06, C12N 5/10

(54) **METHOD OF TESTING REMEDY OR PREVENTIVE FOR HYPERLIPEMIA**

(30) Priority: 09.12.1999 JP 34997699
(71) Applicant: Sankyo Company, Limited, Tokyo 103-8426 (JP)
(72) Inventor: KOISHI, Ryuta, Shinagawa-ku, Tokyo 140-8710 (JP); ANDO, Yosuke, Shinagawa-ku, Tokyo 140-8710 (JP); ONO, Mitsuru, Shinagawa-ku, Tokyo 140-8710 (JP); FURUKAWA, Hidehiko, Shinagawa-ku, Tokyo 140-8710 (JP); HORIKOSHI, Hiroyoshi, Shinagawa-ku, Tokyo 140-8710 (JP); FUJIWARA, Toshihiko, Shinagawa-ku, Tokyo 140-8710 (JP)
(74) Representative: Gibson, Christian John Robert
(86) International application number: JP0008722
(87) International publication number: WO01042499

(57) **Abstract**

The present invention provides a novel method for testing a therapeutic or preventive agent for hyperlipidemia, and a nucleic acid probe, a primer, and an antibody which are used in the method.

Specifically there can be provided a method for testing the effect of a test substance as a therapeutic or preventive agent for hyperlipidemia by expression of a gene shown in SEQ ID No. 1 and SEQ ID No. 2 of the Sequence Listing which participates in regulation of neutral-fat concentration in the blood of a mammal, and a nucleic acid probe, primer or antibody used for the method.
According to the method of the present invention, search is possible of a candidate substance for a therapeutic or preventive agent for hyperlipidemia.

## Description

### [Field of the Invention]

The present invention relates to a novel method for testing a therapeutic or preventive agent for hyperlipidemia, and a nucleic acid probe, a primer and an antibody which are used in the method.

### [Background of the Invention]

The number of patients suffering from hyperlipidemia has been significantly increased, as a result of superfluous ingestion of fat and cholesterol accompanying change of recent eating habits. Hyperlipidemia is a disorder wherein concentration of serum lipids, i.e., cholesterol, neutral fat (triglyceride, TG), phospholipid, free fatty acids or the like in the serum gets high, and is a serious risk factor of arteriosclerosis. Furthermore, the possibility of it causing complications, such as hypertension, angina or myocardial infarction which are associated with coronary arteriosclerosis, and cerebral infarction gets high.

Although many compounds having an anti-hyperlipidemic action have been reported, many of them have various unavoidable side effects at present when they are used continuously, since they are chemically synthesized.

On the other hand, pravastatin sodium which is now commercially available is one of several strong antilipidemic agents originating from microbial metabolites, and acts as a repressor of the HMG-CoA reductase which is a rate limiting enzyme in the cholesterol biosynthesis system. Thus, if a gene encoding a protein relevant to hyperlipidemia which exists in a living body could be identified, more effective antilipidemic agent having no (or few) side effect could be developed by inhibiting a function thereof directly or controlling expression thereof. However, such a gene which is closely related to the onset of hyperlipidemia has not been known.

On the other hand, the same sequence as the nucleic acid probe used in a method of the present invention is disclosed on the Genbank database as a nucleotide sequence which encodes "angiopoietin-related protein 3", and the same nucleotide sequence is disclosed as a sequence which encodes "zalpha5" in International patent publication No. WO 99/55869. However, the relationship between the genes having these nucleotide sequences and hyperlipidemia has not been known at all. Therefore, it has not been known that a part of the nucleotide sequence which encodes "the angiopoietin related protein 3" or "zalpha5" is useful for testing a therapeutic or preventive agent for hyperlipidemia.

Namely, the purpose of the present invention is to provide a novel method for testing a therapeutic or preventive agent for hyperlipidemia and a nucleic acid probe, a primer, or an antibody used in the method.

### [Disclosure of the invention]

The inventors of the present invention narrowed down the position of the gene causing hyperlipidemia on the chromosome by comparing the genes of an inherently hypolipidemic mouse with the genes of a hyperlipidemic mouse, in order to search for the target gene of a therapeutic or preventive agent for hyperlipidemia. Then, they succeeded in specifying the gene which is highly expressed in a hyperlipidemic mouse. As a result of homology comparison, it has been confirmed that cDNA originating from this gene has been disclosed as a nucleotide sequence which encodes "angiopoietin related protein 3" in the Genbank database. However, the inventors of the present invention have found that the concentration of neutral fat in blood rises when the gene having this nucleotide sequence in a hypolipidemic mouse is forcibly expressed, and confirmed that the gene has a new function which has not been reported until now. Then, they succeeded in developing a new method for testing a therapeutic or preventive agent for hyperlipidemia which uses a detection system of gene expression using the nucleotide sequence or a part thereof as a probe or a primer. Moreover, they prepared an antibody specific to the polypeptide encoded by the nucleotide sequence, and developed a method for testing a therapeutic or preventive agent for hyperlipidemia using an experimental system which detects the amount of production of the polypeptide using the antibody. Furthermore, they produced an animal model by introducing the nucleotide sequence into a laboratory animal, and succeeded in developing a novel method for testing a therapeutic or preventive agent for hyperlipidemia using the animal model. Thereby, they have completed the present invention.

First, the present invention relates to a method for testing an effect of a substance as a therapeutic or preventive agent for hyperlipidemia comprising the following steps:
1) culturing cells in the presence or absence of a test substance;
2) detecting the amount of expression of mRNA which has a nucleotide sequence of any one of the following sequences a) to e) (however, t in the sequence is read as u) in the cultured cells obtained in the above l):
   a) the nucleotide sequence shown in the nucleotide numbers 47-1411 of SEQ ID No. 1 of the Sequence Listing;
   b) the nucleotide sequence shown in the nucleotide numbers 78-1457 of SEQ ID No. 3 of the Sequence Listing;
   c) the nucleotide sequence of DNA incorporated into a phagemid carried by transformed Escherichia coli E.coli pBK/m55-1 SANK72199 (FERM BP-6940);
   d) the nucleotide sequence of DNA incorporated into a phagemid carried by transformed Escherichia coli E.coli pTrip/h55-1 SANK72299 (FERM BP-6941);
   e) the nucleotide sequence which hybridizes with the polynucleotide consisting of an antisense sequence of the nucleotide sequence described in any of the above a) to d) under stringent conditions, and encodes a polypeptide having the activity of raising neutral fat concentration in blood; and
3) comparing the amount of expression of mRNA between the cells cultured in the absence of the substance and the cells cultured in the presence of the substance, as a result of detection in the above step 2). The above-mentioned cultured cells preferably originate from the liver (primary-culture hepatocytes or the like), and desirably originate from primates or rodents. More preferably, they are cells from human, mouse, rat or hamster sources, but the present invention is not limited to them.

In the above-mentioned method, the method for detecting the amount of mRNA expressed is preferably Northern blot, dot blot or a slot blot, RT-PCR, a ribonuclease protection assay, a run-on assay, DNA chip analysis, DNA micro array analysis, or the quantum PCR method, but the present invention is not limited to them.

Secondly, the present invention relates to a nucleic acid probe which may be used in the above-mentioned method, i.e., a polynucleotide having a nucleotide sequence hybridizing to mRNA containing the nucleotide sequence described in any of the above a) to d) (however, t in the sequence is read as u) under stringent conditions [excluding those containing the nucleotide sequences disclosed in the following a) to d)]. Moreover, there can also be used preferably as a nucleic acid probe in the above-mentioned method: DNA consisting of at least 15 nucleotides of the nucleotide sequence shown in the nucleotide numbers 47-1411 of SEQ ID No. 1 of the Sequence Listing wherein one or more of nucleotides are deleted at one end or both ends thereof, or DNA consisting of at least 15 nucleotides of the nucleotide sequence shown in the nucleotide numbers 78-1457 of SEQ ID No. 3 of the Sequence Listing wherein one or more of nucleotides are deleted at one end or both ends.

Thirdly, the present invention relates to a method for testing the effect of a substance as a therapeutic or preventive agent for hyperlipidemia comprising the following steps:
1) culturing cells in the presence or absence of the test substance;
2) detecting the amount of production of polypeptide having an amino acid sequence encoded by the nucleotide sequence of any one of the above a) to e) or a part thereof in the supernatant of the cultured cells obtained in the above l) using an antibody specifically recognizing the polypeptide; and
3) comparing the amount of production of the polypeptide between the cells cultured in the absence of the substance and the cells cultured in the presence of the substance, as a result of the above mentioned step 2); and to an antibody used in the above step 2). The antibody which specifically recognizes a polypeptide consisting of the amino acid sequence encoded by any one of the above-mentioned nucleotide sequences a) to e) or a part thereof is preferably an antibody which specifically recognizes a polypeptide which consists of the amino acid sequence shown in the amino acid number 17-455 of SEQ ID No. 2 of the Sequence Listing or a part thereof, a polypeptide which consists of the amino acid sequence shown in 19-455 of the same sequence as the above or a part thereof, or a polypeptide which consists of the amino acid sequence shown in the amino acid number 17-460 of SEQ ID No. 4 of the Sequence Listing or a part thereof, and furthermore, it is preferably those recognizing specifically the amino acid sequence shown in the amino acid sequence shown in the amino acid numbers 1-13 of SEQ ID No. 9 of the Sequence Listing or the amino acid numbers 1-14 of SEQ ID No. 10.

Moreover, as the method for detecting an antibody in the above-mentioned process 2), Western blotting, a dot blot or a slot blot, a solid-phase enzyme immunoassay (the ELISA method), or a radioisotope immunoassay (the RIA method) is preferable, but the present invention is not limited to them.

The forth aspect of the present invention relates to a kit for testing a therapeutic or preventive agent for hyperlipidemia which comprises the above-mentioned antibody.

The fifth aspect of the present invention relates to a method for testing an effect of a substance as a therapeutic or preventive agent for hyperlipidemia comprising the following steps:
1) administrating a substance to be tested to an animal other than a human obtained by genetic manipulation in which a foreign gene containing the nucleotide sequence shown in any one of the above a) to e) is introduced so that the gene can be highly expressed;
2) measuring the concentration of neutral-fat in the blood of the animal shown in l).

In this method, the animal other than a human is preferably a mouse, but the present invention is not limited thereto. Moreover, the method for introducing a foreign gene to a non-human animal is preferably a method of infecting the animal with an adenovirus which contains an adenovirus vector in which the gene is introduced, but the present invention is not limited thereto.

The sixth aspect of the present invention relates to DNA or RNA which consists of an antisense sequence of the nucleotide sequence which consists of a continuous 15 to 30 nucleotides in the nucleotide sequence shown in the nucleotide numbers 78-1457 of SEQ ID No. 3 of the Sequence Listing, or an antilipidemic agent which contains the DNA or RNA as an active ingredient

Namely, the present invention provides a method for testing an effect of a substance as a therapeutic or preventive agent for hyperlipidemia by expression of the gene which participates in the regulation of neutral-fat concentration in the blood of mammals and has a nucleotide sequence shown in SEQ ID No. 1 or SEQ ID No. 3 of the Sequence Listing as an index; a nucleic acid probe, a primer and an antibody used in the method; and an antisense nucleic acid molecule of DNA which has the nucleotide sequence shown in SEQ ID No. 3 of the Sequence Listing.

In the present invention, the word "hybridizes under stringent conditions" means that it hybridizes at 68 °C in a commercially available hybridization solution ExpressHyb Hybridization Solution (manufactured by Clontech), or it hybridizes under conditions equivalent thereto.

Specifically, the method of the present invention comprises: measuring expression of the gene having a nucleotide sequence shown in the nucleotide numbers 47-1411 of SEQ ID No. 1 of the Sequence Listing, or the nucleotide sequence shown in the nucleotide numbers 78-1457 of SEQ ID No. 3 of the Sequence Listing, or a gene encoding a polypeptide having the same activity as the polypeptide encoded thereby, by detecting specifically the nucleic acid (mRNA) or the polypeptide, and selecting the substance which reduces the expression amount of the gene as a candidate substance for a therapeutic or preventive agent for hyperlipidemia. Hereafter, the present invention will be explained.
(A) Detection of nucleic acid
   1) Probe
      The probe used in the method of using nucleic acid hybridization among embodiments for detecting a nucleic acid is DNA or RNA wherein the nucleotide sequence thereof is that hybridizing with a polyribonucleotide having any one of the following sequences a) to e) (t in the sequence is read as u) under stringent conditions:
      a) the nucleotide sequence shown in the nucleotide numbers 47-1411 of SEQ ID No. 1 of the Sequence Listing;
      b) the nucleotide sequence shown in the nucleotide numbers 78-1457 of SEQ ID No. 3 of the Sequence Listing;
      c) the nucleotide sequence of the DNA fragment incorporated into a phagemid carried by transformed Escherichia coli E.coli pBK/m55-1 SANK72199 (FERM BP-6940);
      d) the nucleotide sequence of the DNA fragment incorporated into a phagemid carried by transformed Escherichia coli E.coli pTrip/h55-1 SANK72299 (FERM BP-6941);
      e) the nucleotide sequence which hybridizes with a polynucleotide having an antisense sequence of the nucleotide sequence described in any one of the above a) to d) under stringent conditions, and has the activity of raising neutral-fat concentration in blood. There can be used any probe that hybridizes to a polynucleotide having the nucleotide sequence described in any one of the above a) to e) under stringent conditions, and enabling the polyribonucleotide to be detected, for example, a polynucleotide having an antisense sequence of the nucleotide sequence described in any one of the above a) to e), a polynucleotide having a partial sequence consisting of at least 15 continuous nucleotides in the antisense sequence, a modified sequence of the antisense nucleotide or the like. Among them, the above-mentioned polynucleotide having an antisense sequence of the nucleotide sequence described in the above a) can be obtained as a labeled probe by directly labeling, according to a well known method, cDNA cloned from a cDNA library originating from a mouse liver based on the nucleotide sequence information shown in SEQ ID No. 1 of the Sequence Listing according to a well known method such as a plaque-hybridization method, a colony-hybridization method, or a PCR method, or by labeling in a replication or transcription reaction by a polymerase reaction using a cDNA clone as a template. The polynucleotide having an antisense sequence of a nucleotide sequence described in b) can be obtained as a labeled probe from a cDNA clone by performing the same operation based on the nucleotide-sequence information shown in SEQ ID No. 3 of the Sequence Listing from a cDNA library originating from human liver. On the other hand, a polynucleotide having the antisense sequence of the nucleotide sequence shown in the above c) or d) can be obtained from the recombinant phagemids carried by a transformed Escherichia coli E.coli pBK/m55-1 SANK 72199 or E.coli pTrip/h55-1 SANK 72299, which was, internationally deposited on November 19, 1999 in Kogyo Gijutsuin Seimei-Kogaku Kogyo Gijutsu Kenkyujo (National Institute of Advanced Industrial Science and Technology, International Patent Depositary) at 1-1-3, Higashi-cho, Tsukuba-shi, Ibaraki-ken, Japan, and were accorded the accession numbers FERM BP-6940 and FERM BP-6941 respectively.

      Furthermore, the polynucleotide which has the antisense sequence of the above-mentioned nucleotide sequence shown in e) can be obtained from a cDNA clone isolated by cloning according to the plaque-hybridization method or the colony-hybridization method from a cDNA library originated from an arbitrary mammalian source (preferably liver) using a polynucleotide which has the antisense sequence of the nucleotide sequences shown in a) to d) obtained as mentioned above as a probe.
      The polynucleotide having a partial sequence consisting of at least 15 continuous nucleotides in the antisense sequence of the nucleotide sequences shown in any one of the above a) to e) can be obtained by chemosynthesis if the polynucleotide consists of a few dozens of nucleotides. Alternatively, it can be obtained by subcloning, by PCR or the like, any one of the partial sequences in a cDNA clone which has the nucleotide sequence of any one of the above a) to e) obtained as mentioned above and then prepared as a probe having an antisense sequence by the same method as the above.
      For example, the polynucleotide having the partial sequence which consists of continuous dozens of nucleotides in the antisense sequence of the nucleotide sequence shown in SEQ-ID-No. 1 of the Sequence Listing wherein several nucleotides are added, deleted, and/or added can also be used in the method of the present invention, as long as it hybridizes with the polyribonucleotide described in any one of the above a) to e) under stringent conditions. Such a polynucleotide can be produced according to a chemosynthesis method or a variation introducing method using enzyme reactions which is well-known in the technical field of the present invention, such as polymerase chain reaction (hereinafter referred to as "PCR").
      Moreover, the probe used in the method of the present invention is not limited to what consists of a single nucleotide sequence. That is, in the method of the present invention, for example, a mixture of two or more kinds of nucleotide sequences which satisfy the above-mentioned requirements, may be used as a probe, or multiplex detection using two or more of these kinds of nucleotide sequences individually, may be performed.
   2) Primer for RT-PCR
      Another embodiment of detection of the nucleic acids in the present invention is a method of amplifying a DNA fragment specifically by PCR after performing a reverse transcriptase reaction using mRNA as a template, so-called RT-PCR. In this method, in order to amplify the target nucleotide sequence specifically, an antisense primer complementary to the specific partial sequence of the intended mRNA and a sense primer complementary to the specific partial sequence of the sequence of cDNA generated with reverse transcriptase from the antisense primer are used.
      The antisense primer used for both a reverse transcriptase reaction and PCR substantially consists of a continuous sequence of at least 18 nucleotides, preferably at least 23 nucleotides in an antisense sequence of the above-mentioned nucleotide sequence shown in any one of the above a) to e).
      On the other hand, the sequence of the sense primer used in PCR consists of arbitrary partial sequences of at least 18 nucleotides, preferably at least 21 nucleotides, upstream of the sequence at the 5'-most end of the sequence corresponding to the complementary strand of the above-mentioned antisense primer in the above-mentioned nucleotide sequences shown in any one of the above a) to e). However, if there are complementary sequences in a sense primer and an antisense primer respectively, there is a possibility of an nonspecific sequence being amplified as a result of the fact that the primers are annealed to each other, thus impeding detection of the specific gene. Therefore, it is preferable to design the primers so that such a combination may be avoided.
      A nucleotide sequence which does not have a relationship with the above-mentioned nucleotide sequences shown in any one of the above a) to e) may be added to these antisense primers and sense primers as a linker at the 5'-end of the nucleotide sequences specified above. However, the linker is preferably one which is not unspecifically annealed with the nucleic acid in a reaction mixture during reaction so that it may not impede detection of the specific gene.
   3) Cells or animals for detection of gene expression
      Cultured cells used in the method of the present invention should just be a mammalian cell in which the gene having the above-mentioned nucleotide sequences shown in any one of the above a) to e) is expressed. Cultured cells originating from mammalian liver (preferably primary-culture hepatocytes) are preferable, but artificially transformed cells, for example, cells to which the gene having the above-mentioned nucleotide sequence shown in any one of the above a) to e) is introduced together with a promoter region (for example, CHO cells) can also be used. As the mammalian source, a human, a mouse, a rat, or a hamster is preferable, and a human or a mouse is more preferable. Moreover, primary-culture hepatocytes of KK mouse (available from Nihon Kurea) which is a hyperlipidemic mouse are still more preferable, without being limited thereto. Moreover, when it is considered that it is more preferable than use of culture cells, it is also possible to adopt a method wherein a test substance is administered to a mammal, and expression of the gene having the above nucleotide sequence shown in any one of a) to e) in the organs or tissue cells extracted from the animal is measured. The organs or tissue in which expression of the gene should be detected may be those wherein the gene having the above nucleotide sequence shown in any one of a) to e) can be expressed, and is preferably liver. A preferable mammal in this embodiment may be a human, a mouse, a rat, or a hamster, and human or a mouse is more preferable. For example, the above-mentioned KK mouse is preferably used as a mouse, but the present invention is not limited thereto.
      The cultured cells used in the method of the present invention can be cultured under any conditions wherein the gene having the above nucleotide sequence shown in any one of a) to e) can be expressed in the case that the test substance is not added. For example, the established culture conditions are known for the culture cells, and the cells may be cultured under the conditions when the cells can express the gene having the above nucleotide sequence shown in any one of a) to e) under said conditions.
   4) Addition of test substance
      A test substance is added to the culture medium during culturing of the above-mentioned cells, and the cells are cultured for a certain period. Examples of a test substance may include: a compound, a microbial metabolite, an extract of a plant or an animal tissue, derivatives thereof, or mixtures thereof or the like. The dose and concentration of the test substance can be suitably determined. Alternatively, for example, a dilution series is prepared and it is used as two or more sorts of dose. The period of culturing in the presence of the test substance may also be suitably determined, and it is preferably from 30 minutes to 24 hours. When the test substance is administrated to a mammal, the dosage form such as oral administration, intravenous injection, intraperitoneal injection, transdermal administration, and a hypodermic injection can be properly used depending on the physical properties of the test substance or the like.
   5) Preparation of a sample
      It is preferable to extract RNA from the cells cultured as mentioned above by dissolving the cells directly in a solvent for RNA extraction (for example, those containing a component which has the activity of inactivating ribonuclease, such as phenol), immediately after the end of culturing. Alternatively, the cells are recovered by a method of scraping the cells carefully with a scraper so that the cells may not be destroyed, or a method of gently dissociating the cells from culture-medium material using proteases, such as trypsin, or the like. Then, they are transferred to an RNA extraction process promptly.
      As a method of extracting RNA, there can be adopted a thiocyanic acid guanidine- cesium chloride ultracentrifugal method, a thiocyanic acid guanidine-hot phenol process, a guanidine hydrochloric acid method, an acidic thiocyanic acid guanidine-phenol-chloroform method (Chomczynski, P. and Sacchi, N. (1987), Anal. Biochem., 162, 156-159), or the like. An acidic thiocyanic-acid guanidine phenol chloroform method is preferable.
      A method of further purifying mRNA from the obtained RNA will be explained below. Since it is known that many mRNAs existing in the cytoplasm of an eucaryocyte have a poly (A) sequence at the 3' end, mRNA can be purified using this property, by adsorbing mRNA to a biotinized oligo (dT) probe, catching the mRNA on a paramagnetic grain to which streptoavidin is fixed using binding between biotin/streptoavidin, and eluting the mRNA after a washing operation. Moreover, there can also be used a method for purification comprising adhering mRNA to an oligo (dT) cellulose column, and then eluting it. Furthermore, the mRNA can also be further fractionalized by sucrose density gradient centrifugation or the like. However, for the method of the present invention, these mRNA purifying processes are not indispensable, and total RNA can also be used for a subsequent process as long as expression of the gene having the nucleotide sequence shown in any one of the above a) to e) can be detected.
   6) Immobilization of a sample
      When detecting according to nucleic acid hybridization, in order to detect specifically the gene in the RNA sample obtained as mentioned above, the RNA sample is subjected to agarose electrophoresis, and then transferred to a membrane for a hybridization experiment (hereinafter referred to as "membrane") (Northern blotting), or immobilized on a membrane by a so-called dot blot method or a slot blot method wherein a sample is directly infiltrated into a direct membrane. As the membrane, there can be used nitrocellulose membrane (for example, High bond-C pure (manufactured by Amersham Pharmacia ) or the like), a positive charge nylon membrane (for example, High bond-N+ (manufactured by Amersham Pharmacia) or the like), or hydrophilic nylon membranes (for example, High bond-N/NX (manufactured by Amersham Pharmacia) or the like).
      An agarose electrophoresis method for Northern blotting can be an agarose formamide gel electrophoresis method, a method of treating a sample with glyoxal and dimethyl sulfoxide to give denaturing and migrated in agarose gel produced with phosphoric-acid buffer solution, an agarose-gel methylmercury electrophoresis method (Maniatis, T. et al. (1982) in "Molecular Cloning A Laboratory Manual" Cold Spring Harbor Laboratory, NY.), or the like, but is not limited thereto.
      As a so-called blotting method for transferring RNA from gel after electrophoresis to a membrane, there can be adopted a capillary transferring method (Maniatis, T. et al. (1982) in "Molecular Cloning A Laboratory Manual" Cold Spring Harbor Laboratory, NY.), a vacuum method, an electrophoresis method (Maniatis and T. et al. (1989) in "Molecular Cloning A Laboratory Manual" 2nd ed. Cold Spring Harbor Laboratory, NY.), or the like. Equipment for the dot blot method or the slot blot method are also commercially available (for example, Biodot (manufactured by Bio-Rad) or the like).
      After the blotting, the RNA transferred to the membrane is fixed thereto (this operation depends on the material of the membrane, and the fixing operation is not necessary for some products).
   7) Labeling of a probe
      In performing detection by nucleic acid hybridization, a labeling method of a probe for detecting a specific mRNA in a RNA sample immobilized as mentioned above and a detection method are described below:
      i) Radioisotope labeling
         A labeled DNA probe is prepared using a DNA fragment or a vector carrying it or the like as a material or template, according to a nick translation, (for example, using a nick-translation kit (manufactured by Amersham Pharmacia ), or the like), a random prime method (for example, using a multi-prime DNA labeling system (manufactured by Amersham Pharmacia) or the like), an end labeling method (for example, using Megalabel (TAKARA SHUZO CO., LTD.), 3'-end labeling kit (manufactured by Amersham Pharmacia), or the like). A labeled RNA probe is prepared according to an in vitro transferring method using SP6 promoter or T7 promoter in a vector in which the DNA to be a template is subcloned. The probes can be detected by radioautography using a X-ray film or an imaging plate, and quantification can also be conducted using densitometry (for example, a GS-700 imaging densitometer (manufactured by Bio-Rad) is used) in the case of the X-ray film, or the BAS2000II (manufactured by Fuji film) system in the case of the imaging plate, respectively.
      ii) Enzyme labeling
         The DNA or RNA fragment is directly labeled with enzyme. Examples of the enzyme used for labeling include: alkaline phosphatase (AlkPhos Direct system for chemiluminescence (manufactured by Amersham Pharmacia ) or the like is used) and Western horseradish peroxidase (ECL direct nucleic acid labeling and detection system (manufactured by Amersham Pharmacia), or the like are used). Detection of the probe is performed by immersing the membrane in a enzyme reaction buffer solution containing a substrate which can make catalytic reaction of the enzyme used for labeling detectable, for example, the substrate generating a colored substance, or the substrate emitting light as a result of the catalytic reaction. When a coloring substrate is used, detection can be visually conducted. When an emitting substrate is used, it is detected by photography using an instant camera, or by radioautography using a X-ray film or an imaging plate in a similar way to the case of a radioisotope labeling. Furthermore, when an emitting substrate is used, the quantification can be conducted using densitometry or the BAS2000II system.
         iii) Labeling by other molecules
            Fluorescein labeling: The DNA fragment is labeled according to a nick translation method, a random prime method, or a 3'-end labeling method (ECL3'-oligo labeling system available from Amersham Pharmacia). RNA is labeled by in vitro transcription using SP6 and T7 promoter;
            Biotin labeling: The 5'-end of the DNA is labeled (using an oligonucleotide biotin labeling kit available from Amersham Pharmacia), or the DNA fragment is labeled according to a nick translation method, a random prime method, or the like;
            Digoxigenin modified dUTP labeling: The DNA fragment is labeled according to a nick translation method, a random prime method, or the like.

         Detection of these labeled molecules comprises the operation of binding a molecule specifically binding to the molecule which was labeled with a radioisotope or an enzyme to a probe. In the case of fluorescein or digoxigenin, the molecule specifically binding is an anti-fluorescein antibody or an anti-digoxigenin antibody. In the case of the biotin, it is avidin or streptoavidin. After binding it to a probe, detection can be conducted using the labeled radioisotope or the enzyme according to the same method as description in the above i) or ii).
   8) Hybridization
      Hybridization may be performed by a well-known method in the technical field of the present invention. The relationship between composition of a hybridization solution or a washing solution, and the hybridization temperature or the washing temperature in the present invention can be defined as described in the reference (baiojikken illustrated 4, p148, Shujunsha), but preferable conditions are as follows:
      Hybridization solution: ExpressHyb Hybridization Solution (manufactured by Clontech);
      Final concentration of the probe (in the case of a radio-labeled probe): 1 to 2 x 10⁶ cpm/ml (preferably 2 x 10⁶ cpm/ml);
      Hybridization temperature and time: 68 °C, 1 to 24 hours.

      Membrane Washing conditions:
      i) A shaking operation is conducted in 0.1 to 5 x SSC (most preferably 2 x SSC), 0.05 to 0.1% SDS (most preferably 0.05%) sodium dodecyl sulfate (hereinafter referred to as "SDS"), at room temperature to 42 °C (most preferably room temperature) for 20 to 60 minutes (most preferably for 20 minutes), twice to six times (most suitably 3 times), with exchange of the washing solution.
      ii) After the operation i), a shaking operation is conducted in 0.1 x SSC, 0.1 % SDS, at 50 to 65 °C for 20 to 60 minutes twice to six times, exchanging the washing solution. Alternatively, the shaking operation is in 0.2 to 0.5 x SSC, 0.1% SDS, at 62 to 65 °C for 20 to 60 minutes twice to six times, with exchange of the washing solution. Most preferably, shaking in 0.1 x SSC and 0.1 % SDS at 50 °C for 20 minutes is performed three times, with exchange of the washing solution.

      After washing, as described in the above 7), detection and quantification suitable for the method of labeling a probe are conducted. Furthermore, the expression amount of a gene wherein it is known that an expression amount per cell is stable (for example, probes for detecting gene expression of 23 kDa highly basic protein, α-tubulin, glyceraldehyde 3-dehydrogenase, hypoxanthine guanine phosphoribosyltransferase, phospholipase A2, ribosomal protein S9, and ubiquitin or the like are commercially available) in each sample is measured simultaneously in order to rectify dispersion resulting from a difference in the amount of RNA between each sample or the like, and then the relative value of the expression amount of the gene to be detected on the basis of the expression amount of this stable expression gene is compared between the cell population to which the test substance is administered and the cell population to which it is not administered. Thereby, more precise evaluation can be performed.
      From the above results, a test substance which lowers the expression amount of a gene having the nucleotide sequence shown in any one of the above a) to e) may serve as a therapeutic or preventive agent of hyperlipidemia.
   9) RT-PCR reaction
      The conditions of each reaction in the embodiment wherein a nucleic acid is detected by RT-PCR will be shown below. Usually, a sample for detection by RT-PCR does not need to be purified to be Poly (A)⁺RNA.
      i) Reverse transcriptase reaction
         Example of composition of a reaction mixture (total amount of 20 µl):
         Total RNA: an adequate amount;
         Magnesium chloride: 2.5 to 5 mM (preferably 5 mM);
         1 x RNA PCR buffer solution (10 mM of Tris hydrochloric acid (pH 8.3 to 9.0 at 25 °C (preferably 8.3)), 50 mM of potassium chloride);
         dNTPs: 0.5 to 1 mM (preferably 1 mM);
         Antisense primer: 1 µM (2.5 µM of a commercial random primer or oligo (dT) primer (12 -20 nucleotides) can also be added as a substitute for an antisense primer);
         Reverse transcriptase: 0.25 to 1 unit/µl (preferably 0.25 unit /µl);
         adjusted to 20 µl with sterilized water.
         Reaction temperature conditions:
         after keeping at 30 °C for 10 minutes (only when using random primers), keep at 42 to 60 °C (preferably 42 °C) for 15 to 30 minutes (preferably for 30 minutes), and then heat at 99 °C for 5 minutes to deactivate the enzyme, and then cool at 4 to 5 °C (preferably 5 °C) for 5 minutes.
      ii) PCR
         Example of reaction-mixture composition:
         Magnesium chloride: 2 to 2.5 mM (preferably 2.5 mM);
         1 x PCR buffer solution (10 mM of Tris hydrochloric acid (pH 8.3 to 9.0 at 25 °C (preferably 8.3)), 50 mM of potassium chloride);
         dNTPs: 0.2 to 0.25 mM (preferably 0.25 mM);
         Antisense primer and sense primer: 0.2 to 0.5 µM (preferably 0.2 µM);
         Taq polymerase: 1 to 2.5 units (preferably 2.5 units);
         The total amount is adjusted to 80 µl with sterilized water, and the total amount is added to the total amount of the reaction mixture wherein a reverse transcription reaction has been terminated, and then PCR is initiated.

      Reaction temperature conditions: heating at 94 °C for 2 minutes first, heating at 90 to 95 °C (preferably 94 °C) for 30 seconds, and then 28 or 50 cycles (preferably 28 cycles) of a temperature cycle which comprises heating at 40 to 60 °C (preferably at a temperature within the range from a dissociation temperature (Tm) calculated from characteristics of a primer to a temperature 20°C lower than it) for 30 seconds and at 70 to 75 °C (preferably 72 °C) for 1.5 minutes, and then cooled at 4 °C.
      After PCR, the reaction mixture is subjected to electrophoresis and it is detected whether a band of the intended size is amplified. In order to perform quantitative detection, PCR is carried out under the same condition using the cDNA clone previously diluted stepwise as a standard template DNA, and the number of temperature cycles which enables quantitative detection is previously defined, or a part of the reaction mixture is sampled every 5 cycles, and each of them is subjected to electrophoresis. Moreover, for example, if radio-labeled dCTPs are used for the PCR reaction, quantification is enabled by measuring radioactivity incorporated in the band as an index.
      The detection results are compared between the sample originating from the cells cultured in the presence of the test substance and the sample originating from the cells cultured in the absence of the test substance. A test substance in which the expression amount of the gene having the nucleotide sequence shown in any one of the above a) to e) is reduced may serve as a therapeutic or preventive agent for hyperlipidemia.
   10) Other methods
      Other methods of measuring the expression amount of the gene having the nucleotide sequence shown in any one of the above a) to e) are mentioned below.
      i) Ribonuclease protection assay (RNase protection assay):
         When a labeled probe is hybridized only to mRNA having the nucleotide sequence shown in any one of the above a) to e) (however, t in the sequence is read as u) to form a double-stranded polynucleotide, and then ribonuclease is added to the sample and incubated, mRNA to which the probe is hybridized will not be digested by the ribonuclease since a double strand is formed, and other RNA will be digested. Thereby, only a double-stranded polynucleotide remains (if a probe is shorter than the mRNA to be detected, the double-stranded polynucleotide corresponding to the chain length of the probe will remain). The expression amount of the target gene is measured by quantifying the double-stranded polynucleotide. Specifically, it is conducted according to the method described below.
         It is preferable to digest a surplus labeled probe with a ribonuclease in order to separate surely the labeled probe which remained without forming a double strand from the double-stranded polynucleotide and to make the quantification easy. However, if a ribonuclease which can also digest single stranded DNA is used, either DNA or RNA can be used as the labeled probe. The method for preparing the labeled probe is similar to the method described above in 1) to 7). The length of the probe used in this method is preferably about 50 to 500 nucleotides. Moreover, a probe wherein strands complementary to each other exist, such as a probe produced by directly labeling the double stranded DNA and thermally denaturing it is not suitable for the method.
         A RNA probe is, for example, prepared according to the following method. A template DNA is first incorporated in a plasmid vector (for example, pGEM-T (Promega) or the like) having bacteriophage promoters (T7, SP6, T3 promoter, or the like). Then, the recombinant plasmid vector is digested with a restriction enzyme at a position just downstream of the insert so that only one part may be cut. In vitro transcription reaction is performed using the acquired straight chain DNA as a template in the presence of the radio-labeled ribonucleotide. Enzymes such as T7, SP6, or T3 polymerase or the like, are used for this reaction depending on the promoter in the vector. The operation described above can be conducted, for example, using Riboprobe system- T7 and -SP6 or -T3 (all of them are manufactured by Promega).
         The steps until an RNA sample is prepared are the same as the above 3) to 5). A ribonuclease protection assay is performed using an equivalent amount to 10 to 20 µg of the prepared total RNA samples and an excess amount corresponding to 5 x 10⁵ cpm of the labeled probe. This operation can be performed using a commercial kit (HybSpeed RPA Kit, manufactured by Anbion). After the resultant sample digested with ribonuclease is subjected to electrophoresis using 4 to 12% polyacrylamide gel containing 8M urea, the gel is dried and subjected to radioautography using a X-ray film. By the above operations, the band of the double-stranded polynucleotide which is not digested with ribonuclease can be detected, and quantified according to the method described in the above i) of 7). Furthermore, if the expression amount of the β-actin gene is measured simultaneously in order to rectify dispersion resulting from differences in the amount of RNA between each sample or the like, more precise evaluation can be performed, as in the case of Northern blotting analysis.
         Thus, the results of detection are compared between the sample originating from the cells cultured in the presence of the test substance, and the sample originating from the cells cultured in the absence of the test substance; and a test substance in which the expression amount of the gene having the nucleotide sequence shown in any one of the above a) to e) is reduced may serve as a therapeutic or preventive agent for hyperlipidemia.
      ii) Run-on assay (See Run-on assay, Greenberg, M. E. and Ziff, E. B. (1984) Nature 311, 433-438 and Groudine, M. et al. (1981) Mol. Cell Biol. 1, 281-288):
         The method is a method of isolating a nucleus from a cell and measuring the transcriptional activity of a target gene. Although it is not a method of detecting mRNA in a cell described above, it is included in "a method of detecting an expression amount of a gene" in the present invention. If a transcription reaction is performed within a test tube using an isolated cell nucleus, only reactions wherein transcription has already started before isolation of the nucleus and the generated mRNA chain elongates will advance. The transcriptional activity of the target gene at the time of isolation of a nucleus can be measured by adding the radio-labeled ribonucleotide during the reaction to label the elongating mRNA, and detecting mRNA hybridizing to the non-labeled probe contained in it. In order to find the time at which the influence of the test substance is the most significantly expressed, the period from addition of the test substance to the culture cells to isolation of the nucleus can be varied. For example, at 30 minutes, 1 hour, 2 hours, 4 hours, 8 hours, and 24 hours after the addition, the nucleus is isolated and subjected to the assay. The specific operation method is approximately the same as those described in the above-mentioned reference, except that non-labeled probe is prepared in a similar way to the description of the above 1). Thus, the results of detection are compared between the sample originating from the cell cultured in the presence of the test substance, and the sample originating from the cell cultured in the absence of the test substance, and the test substance which reduces the transcriptional activity of a gene having the nucleotide sequence shown in any one of the above a) to e) may serve as a therapeutic or preventive agent for hyperlipidemia.
      iii) DNA chip analysis, DNA micro array analysis
         [1] Preparation of a sample for obtaining a probe
            First, mRNAs of the sample originating from the cells cultured in the presence of the test substance, and the sample originating from the cells cultured in the absence of the test substance are extracted and purified. The process until the RNA sample is prepared is the same as described in the above 3) to 5).
         [2] Labeling of a probe
            Although a total RNA that is not purified can also be used as a start material for producing the probe for DNA chip analysis or DNA micro array analysis, it is more preferable to use Poly (A)⁺ RNA purified by the method described in the above 5).
            The method of labeling a probe and the detection method for detection by nucleic acid hybridization will be explained below.
            A probe for analysis using a DNA chip manufactured by Affymetrix: a cRNA probe labeled with biotin is used according to a protocol appended to the DNA chip manufactured by Affymetrix.
            A probe for analysis using DNA micro array: a cDNA is fluorescently labeled by adding d-UTP labeled with fluorochromes (for example, Cy3, Cy5, or the like) or the like when cDNA is prepared from Poly (A)⁺ RNA according to a reverse transcription reaction. At this time, if the sample originating from the cells cultured in the presence of the test substance, and the sample originating from the cells cultured in the absence of the test substance are labeled with pigments different from each other, they can be mixed and used in the later hybridization process.
            A probe for analysis using a membrane filter:
            When cDNA is prepared from Poly (A)⁺ RNA according to a reverse transcription reaction, the probe is labeled by adding d-CTP labeled with a radioisotope (for example, ³²P, ³³P) or the like.
         [3] Immobilized sample
            The immobilized samples for allowing to hybridize to the labeled probe obtained in the above-mentioned process [2] are exemplified as follows.
            A gene chip on which an antisense oligonucleotide synthesized based on an EST (expressed sequence tag) sequence or mRNA sequence on a database is immobilized (manufactured by Affymetrix) (Lipshutz, R. J. et al. (1999) Nature genet. 21, supplement, 20-24):
            The above-mentioned EST sequences or mRNA sequences are most preferably those originating from the same animal as that used for preparation of the probe, but are not limited thereto. For example, those originating from an animal which is closely related thereto can also be used. However, a gene having the nucleotide sequence shown in any one of the above a) to e), a gene currently disclosed on the Genbank database as a nucleotide sequence encoding "angiopoietin related protein 3", or a sequence including the partial sequence of any one of them should be immobilized.
            The DNA micro array or the membrane filter on which a cDNA or a RT-PCR product produced from the mRNA obtained from the cells isolated from the internal organs (preferably liver) of an animal which is the same as or closely related to the animal used for the preparation of the probe is immobilized:
            The cDNA or the RT-PCR product is cloned by carrying out a reverse transcriptase reaction or PCR using the primer produced based on the sequence information of an EST database of the animal used as the material for the mRNA. As a material for preparing a sample, there can be used the cells (preferably of liver origin) expressing a gene having the nucleotide sequence shown in any one of the above a) to e), or a gene currently disclosed on the Genbank database as a nucleotide sequence encoding "angiopoietin related protein 3". The cDNA or the RT-PCR products include those prepared by previously selecting mRNA having a different expression amount using the subtraction method (Diatchenko, L. et al. (1996) Proc. Natl. Acad. Sci. U.S.A. 93, 6025-6030), the differential displaying method (Kato, K. (1995) Nucleic Acids Res. 23, 3685-3690) or the like. Moreover, as a DNA micro array and a filter, there can be used a commercially available one comprising a gene to be detected, namely a gene disclosed on the Genbank database as a nucleotide sequence encoding "angiopoietin related protein 3". Alternatively, the DNA micro array or the filter on which the gene having the nucleotide sequence shown in the above a) to e) is immobilized using a spotter can also be produced (for example, TAKARA SHUZO CO., LTD., GMS417 arrayer, or the like).
            The probes prepared in the above [2] are allowed to hybridize to this immobilized sample separately under the same conditions, or simultaneously as a mixture (Brown, P.O. et al. (1999) Nature genet. 21, supplement, 33-37).
         [4] Analysis
            In the case of analysis using the DNA chip manufactured by Affymetrix:
            According to the protocol attached to the DNA chip manufactured by Affymetrix, hybridization and analysis are performed.
            In the case of analysis using a DNA micro array:
            For example, in the case that a commercial DNA micro array of TAKARA SHUZO CO., LTD. is used, hybridization and washing are performed according to the protocol of the company, and a fluorescent signal is detected with a fluorescence detection apparatus (for example, GMS418 array scanner (TAKARA SHUZO CO., LTD.) or the like) followed by analysis.
            In the case of analysis using a filter:
            Hybridization may be performed by a well-known method in the technical field of the present invention. For example, hybridization and washing are performed, followed by analysis using analysis equipment (for example, Atlasimage (manufactured by Clontech)).
            In any cases described above, the probe in the sample originating from the cells cultured in the presence of the test substance, and the probe in the sample originating from the cells cultured in the absence of the test substance are allowed to hybridize to the immobilized sample of the same lot. At this time, the conditions of hybridization other than the probe to be used are to be the same. As indicated in the above [2], when each probe is labeled with a different fluorochrome, a mixture of both probes can be hybridized to one immobilized sample (Brown, P.O. et al. (1999) Nature genet. 21, supplement, 33-37).
            The amounts of the probes which hybridize to the target gene of an immobilized sample, i.e., a gene having the nucleotide sequence shown in any one of the above a) to e), or a gene having a nucleotide sequence encoding "angiopoietin related protein 3" between the probe in the sample originating from the cells cultured in the presence of the test substance and the probe in the sample originating from the cells cultured in the absence of the test substance are measured as a result of the analysis. As a result, as for the amounts of the probes hybridized to the target gene, if the amount of the probe in the sample originating from the cells cultured in the presence of the test substance is less than the amount of the probe in the sample originating from the cells cultured in the absence of the test substance, the substance suppresses expression of the gene having the nucleotide sequence shown in any one of the above-mentioned a) to e), and thus it can be a therapeutic or preventive agent.
      iv) Others
         The following methods can be mentioned as a method of detecting the test substance which reduces the expression amount of a gene having the nucleotide sequence shown in any one of the above a) to e) compared with that in the sample originating from the cells cultured in the absence of the test substance.
         Namely, there can be used a technique known as "Taqman" wherein PCR and hybridization probing (hereinafter referred to as "probing") are combined in a single reaction (Holland, P. M. et al. (1991) Proc. Natl. Acad. Sci. USA 88, 7276-7280) or the technique wherein PCR and probing are combined in a single reaction (Higuchi et al., Biotechnology, 10, 413-417 (1992)). In the latter method, ethidium bromide which is a nucleic acid detection reagent that emits fluorescence when excited by ultraviolet rays is added to a PCR reaction mixture. Since the fluorescence of ethidium bromide increases in the presence of double stranded DNA, an increase in the fluorescence detected when excitation light is irradiated may be correlated with an accumulation of a double stranded PCR product.
         Furthermore, a method described in the Europe patent application publication No. 0601889 can also be used as another method wherein amplification by PCR and probing are combined. Furthermore, it is also possible to quantify the amount of mRNA(s) using the Light cycler system (manufactured by Roche diagnostics, see Japanese patent application laid-open publication (KOKAI) No. 2000-312600).
(B) Detection of the polypeptide
   As another embodiment of the method of the present invention there is a method of detecting a polypeptide encoded by the gene which is to be detected in the above-mentioned embodiment for detecting gene expression. In this embodiment, a polypeptide in a sample is immobilized to the bottom of a well of 96 well plate, a membrane, or the like, and then detection using an antibody specifically recognizing the target polypeptide is performed. Among them, the method using a 96 well plate is generally a method called solid-phase enzyme immunoassay (ELISA method) or a method called radioisotope immunoassay (RIA method). As a method for immobilizing to a membrane, there are mentioned a method of transferring a polypeptide to a membrane through polyacrylamide gel electrophoresis of a sample (Western blotting) or a so-called dot blot method and a slot blot method wherein a sample or its diluent is directly infiltrated into a membrane.
   1) Preparation of a sample
      Conditions for the kind of cultured cells used in the above-mentioned embodiment of detecting a polypeptide are the same as those in the case of the above-mentioned embodiment for detecting gene expression. Moreover, there can also be adopted a method wherein a test substance is administrated to a mammal, and serum extracted from the animal is used as the sample. The preferable mammal in this case is a human, a mouse, a rat, or a hamster, and more preferably a human or a mouse. For example, although a KK mouse which is a hyperlipidemic mouse is preferably used as a mouse, the present invention is not limited thereto. As for the conditions for culturing cells, the methods of administrating the test substance are also the same as those in the case of the embodiment of detecting gene expression. A test substance the activity of which is to be tested as a therapeutic or preventive agent for hyperlipidemia can be a compound, a microbial metabolite, an extract from a plant or animal tissue, derivatives thereof, or mixtures thereof.
      As a material for preparing the sample for this embodiment, there can be used a culture supernatant or a cytoplasmic fraction of cells cultured in the presence or absence of the test substance; preferably the culture supernatant is used. The culture supernatant is collected after completion of culturing, subjected to filter filtration sterilization treatment, and then used for preparation of a sample for ELISA/RIA or Western blotting.
      As a sample for ELISA/RIA, there can be used, for example a collected culture supernatant as it is, or those suitably diluted with a buffer solution.
      A preparation method of a sample for Western blotting (for electrophoresis) is as follows. First, protein is settled, for example by subjecting a culture supernatant to trichloroacetic-acid treatment, and the precipitate is obtained by centrifugal separation. The precipitate is washed with acetone cooled with ice, air-dried and dissolved in a sample buffer solution containing 2-mercaptoethanol for SDS-polyacrylamide gel electrophoresis (manufactured by Bio-Rad or the like).
      In the case of a dot/slot blot method, the collected culture supernatant itself or those suitably diluted with a buffer solution is directly adsorbed on a membrane, for example using blotting equipment.
   2) Immobilization of a sample
      The sample is immobilized in order to detect specifically a polypeptide in the sample obtained as mentioned above. As a membrane used for Western blotting, the dot blot method or the slot blot method, there can be used nitrocellulose membranes (for example, manufactured by Bio-Rad or the like), nylon membranes (for example, High bond- ECL (Amersham Pharmacia) or the like), cotton membranes (for example, Blot absorbent filter (manufactured by Bio-Rad) or the like), or poly vinylidene difluoride (PVDF) membranes (for example, manufactured by Bio-Rad or the like).
      As a so-called blotting method which is a method of transferring a polypeptide from a gel to a membrane after electrophoresis, there can be used a wet style blotting method (CURRENT PROTOCOLS IN IMMUNOLOGY volume 2 ed by J. E. Coligan, A. M. Kruisbeek, D. H. Margulies, E. M. Shevach, and W. Strober), a semi-dry style blotting method (see the above-mentioned CURRENT PROTOCOLS IN IMMUNOLOGY volume 2), or the like. Equipment for the dot blot method or the slot blot method is also commercially available (for example, Biotechnology dot (manufactured by Bio-Rad) or the like).
      In order to perform detection and quantification by the ELISA method/the RIA method, a sample or a diluent thereof (for example, diluted with phosphoric-acid buffered physiological saline (hereinafter referred to as "PBS") which contains 0.05 % sodium azide) is put into a 96 well plate of exclusive use (for example, Immunoplate maxi soap (manufactured by Nunc) or the like), and left to stand at 4 °C to room temperature over night or at 37 °C for 1 to 3 hours, to immobilize the polypeptide on the bottom of the well.
   3) Antibody
      The antibody used for this embodiment is one that specifically recognizes the polypeptide produced when a gene having a nucleotide sequence described in the above a) to e) is expressed in animal cells, preferably the polypeptide which consists of an amino acid sequence shown in the amino acid number 17-455 (preferably 19-455) of SEQ ID No. 2 of the Sequence Listing or a part thereof, or the polypeptide which consists of an amino acid sequence shown in the amino acid number 17-460 of SEQ ID No. 4 or a part thereof. These antibodies are preferably, for example, an antibody which binds to any of the polypeptides which consist of an amino acid sequence shown in the amino acid number 17-455 (preferably this 19-455) of SEQ ID No. 2 of the Sequence Listing, and the polypeptide which consists of an amino acid sequence shown in the amino acid number 17-460 of SEQ ID No. 4, but does not bind to any other protein originating from mice or human.
      The antibody for this embodiment can be obtained by immunizing an animal with the protein that is to be the antigen or an arbitrary polypeptide having a sequence which is chosen from the amino acid sequence using a conventional method (for example, Shin-seikagaku jikkenkouza 1, protein 1, p.389-397, 1992), and then extracting and purifying the antibody produced in the body. Moreover, a monoclonal antibody can also be obtained by preparing a hybridoma by cell fusion of an antibody-producing cell which produces the antibody to the protein of the present invention and a myeloma cell according to a known method (for example, Kohler and Milstein, Nature 256, 495- 497, 1975, and Kennet, R. ed., Monoclonal Antibody p.365- 367, 1980, Prenum Press, N.Y.).
      The antigen for producing the antibody used for this embodiment can be a polypeptide which consists of the amino acid number 17-455 (preferably 19-455) of SEQ ID No. 2 of the Sequence Listing or a polypeptide consisting of a partial sequence of at least six continuous amino acids thereof, a polypeptide which consists of an amino acid sequence shown in the amino acid number 17-460 of SEQ ID No. 4 or a polypeptide which consists of a partial sequence of at least six continuous amino acids thereof, or a derivative wherein arbitrary amino acid sequences or carriers are added thereto. Preferably, it is one obtained by fusing a Keyhole limpet hemocyanin as a carrier to the C-terminus of a polypeptide which consists of an amino acid number 1-14 of SEQ ID No. 9 of the Sequence Listing or to the N-terminus of a polypeptide which consists of an amino acid sequence shown in the amino acid number 1-14 of SEQ ID No. 10.
      The polypeptide which consists of an amino acid sequence shown in the amino acid number 17-455 (preferably 19-455) of SEQ ID No. 2 of the Sequence Listing, or the polypeptide which consists of an amino acid sequence shown in the amino acid number 17-460 of SEQ ID No. 4 can be obtained by making a host cell produce the polypeptide encoded by the nucleotide sequence shown in the nucleotide number 47-1411 of SEQ ID No. 1 of the Sequence Listing or the nucleotide sequence shown in the nucleotide number 78-1457 of SEQ ID No. 3 of the Sequence Listing by genetic manipulation. Specifically, the host cell of other procaryotes or eucaryotes can be transformed by incorporating DNA having the above-mentioned nucleotide sequence into suitable vector DNA. It is possible to express the gene in each host by introducing a suitable promoter and a sequence inducing gene expression to these vectors.
      Examples of a procaryotic cell for use as a host include Escherichia coli, Bacillus subtilis or the like. In order to transform these host cells with the target gene, the host cell is transformed with a plasmid vector containing a replicon, namely a replication origin, originating from a species compatible with the host and a regulatory sequence. As the vector, a vector comprising a sequence affording selectivity of phenotype to a transformed cell is preferable.
      For example, K12 or the like is well used as Escherichia coli and the plasmid pBR322 and pUC system is generally used as a vector, but it is not limited thereto, and other known strains and vectors can be used. As a promoter in Escherichia coli, there can be used a tryptophan (trp) promoter, a lactose (lac) promoter, a tryptophan lactose (tac) promoter, a lipoprotein (lpp) promoter, a polypeptide-chain tension factor Tu (tufB) promoter, or the like. Any one of these promoters can be used for production of the target polypeptide.
      As a Bacillus subtilis, for example, 207-25 strain is preferable, and pTUB228 (Ohmura, K. et al.(1984) J. Biochem. 95, 87-93) or the like is used as a vector, but it is not limited thereto. A secretion expression out of the cell is also attained by connecting the DNA sequence encoding a signal peptide sequence of the α-amylase of bacillus subtilis.
      Examples of a eucaryotic host cell include: cells of a vertebrate, an insect, and yeast or the like. Examples of the cells of a vertebrate cell include: COS cells (Gluzman, Y. (1981) Cell 23, 175-182, ATCC CRL-1650) which are the cells of an ape, a dihydrofolic acid reductase deficient strain of a Chinese hamster ovary cell (CHO cell, ATCC CCL-61), or the like, but it is not limited thereto (Urlaub, G. and Chasin, L.A. (1980) Proc. Natl. Acad. Sci. USA 77, 4126-4220).
      Expression promoters for vertebrate cells can be those having a promoter upstream of the gene to be expressed, an RNA splicing site, a polyadenylation site and furthermore having a replication origin if desired. Examples of the expression vector include pSV2dhfr (Subramani, S. et al. (1981) Mol. Cell. Biol. 1, 854-864) or the like, but is not limited thereto.
      When COS cells are used as a host cell, expression vectors suitably comprise the SV40 replication origin in COS cells, enabling autonomous replication, a transcription promoter, a transcription termination signal and an RNA splicing site. The expression vectors can be used to transform the COS cells by a known method, such as a diethylaminoethyl (DEAE)-dextran method [cf. Luthman. H, and Magnusson. G. (1983), Nucleic Acids Res., 11, 1295-1308], a phosphate calcium-DNA co-precipitation method [Graham, F. L. and Van der Eb, A. J., (1973), Virology, 52, 456-457] and an electric pulse electroporation method [cf. Neumann, E., et. al., (1982), EMBO J, 1, 841-845] or the like. In the case that a CHO cell is used as the host cell, the transformed cells stably producing the protein of the present invention can be produced by co-transfecting with an expression vector and a vector which can express the neo gene which functions as an antibiotics G418 resistance marker, for example, pRSVneo (Sambrook, J. et al. (1989): "Molecular Cloning A Laboratory Manual" Cold Spring Harbor Laboratory, NY), pSV2-neo (Southern, P. J. and Berg, P. (1982) J. Mol. Appl. Genet. 1, 327-341) or the like, and selecting G-418 resistant colonies.
      In the case that an insect cell is used as a host cell, a strain cell line (Sf-9 or Sf-21) originating from the ovarian-cells of Spodoptera frugiperda of the Lepidoptera Phalaenidae, High Five cells originating from the ootid of Trichoplusiani (Wickham, T. J. et al. (1992) Biotechnol. Prog. I: 391-396 or the like) are often used as a host cell. pVL1392/1393 using a polyhedrin protein promoter of autograph polyhedron virus (AcNPV) is often used as baculovirus transfer vector (Kidd, I. M. and V. C. Emery (1993) The use of baculoviruses as expression vectors, Applied Biochemistry and Biotechnology 42,137-159). Furthermore, a vector using a promoter of baculovirus P10 or a basic protein can also be used. Furthermore, it is possible to express recombinant protein as a secretory protein by fusing the secretion-signal sequence of the envelope surface protein GP67 of AcNPV to the N-terminus of the target protein (Zhe-mei Wang, et al. (1998) Biol. Chem., 379, 167-174).
      As an expression system using a eukaryotic microorganism as a host cell, yeast is generally known, and Saccharomyces yeasts, for example, baker's yeast Saccharomyces cerevisiae and the petroleum yeast Pichia pastoris are preferable. As an expression vector for eukaryotic microorganisms such as yeasts, the promoter of an alcohol dehydrogenase gene (Bennetzen, J. L. and Hall, B. D. (1982) J. Biol. Chem. 257, 3018-3025), the promoter of an acid-phosphatase gene (Miyanohara, A. et al. (1983) Proc. Natl. Acad. Sci. USA 80, 1-5), or the like can be used preferably. In order to express as secretory protein, it is possible to be also expressed as a recombinant which has a secretion signal sequence and the cleavage site of the mature endogenous protease which a host cell has or a known protease in N-terminus. For example, it is known that active form tryptase will be secreted in a medium by fusing the secretion-signal sequence of alpha factor of yeast and the cleavage site of KEX2 protease of petroleum yeast at the N-terminal end and then expressing them in a system where human mast cell tryptase of a trypsin type serine protease is expressed in petroleum yeast (Andrew, L. Niles, et al. (1998) Biotechnol. Appl. Biochem. 28,125-131).
      Transformants obtained by the above methods can be cultured using conventional methods, and thereby protein of the present invention can be produced either intra- or extra- cellularly. Suitable culture media include various commonly used media, depending on the host chosen. For example, for COS cells, there can be used RPMI-1640 and Dulbecco's Modified Eagle's Medium (hereinafter referred to as DMEM), which can be supplemented with, as desired, serum component such as fetal bovine serum.
      The recombinant protein expressed intra- or extra- cellularly by the transformants as described above may be isolated and purified by various well known methods of separation according to the physical and chemical properties of the protein. Suitable specific methods of separation include: treatment with commonly used precipitating agents for protein; various methods of chromatography such as ultrafiltration, molecular sieve chromatography (gel filtration), adsorption chromatography, ion exchange chromatography, affinity chromatography and high performance liquid chromatography (HPLC), dialysis and combinations thereof. Moreover, it can be purified efficiently in a nickel affinity column by fusing six histidine residues with the expressed recombinant protein. The polypeptide of the present invention can be easily manufactured in large quantities in high yield and high purity by combining the above-mentioned methods.
      The antibody obtained as mentioned above can be used for various immunoassays such as the RIA method, the ELISA method, a fluorescent antibody technique, and the passive-hemagglutination reacting method, immunity tissue dyeing, or the like.
   4) Detection
      The antibody obtained by the method of the above 3) is directly labeled, or used for detection as a primary antibody in cooperation with a labeled secondary antibody which recognizes this antibody specifically (recognizes an antibody of the animal used for production of the antibody).
      Although the substance preferable for labeling is an enzyme (alkaline phosphatase or Western horseradish peroxidase) or a biotin (however, the operation of binding enzyme-labeled streptoavidin to the biotin as a secondary antibody is added further), it is not limited thereto. Previously labeled antibodies (or streptoavidin) for the method of using a labeled secondary antibody (or labeled streptoavidin) are commercially available. In the case of RIA, measurement is performed by a liquid scintillation counter or the like using antibodies labeled with a radioisotope such as I¹²⁵ or the like.
      The quantity of the polypeptide which is an antigen is measured by detecting the activity of these enzymes used for labeling. As for alkaline phosphatase or Western horseradish peroxidase, substrates which are colored or emit light according to catalytic action of these enzymes are commercially available.
      When a substrate which is colored is used, it can be detected visually in a Western blot technique or a dot/slot blot method. In the ELISA method, a quantification is preferably carried out by measuring the extinction coefficient (wavelength for measurement depends on the substrate) in each well using a commercial microplate reader. Moreover, it is possible to quantify the antigen concentration in other samples by preparing a dilution series of the antigen preferably used in the above 3) for antibody production, and using it as a standard antigen sample and detecting it together with other samples at the same time to make a standard curve wherein the standard antigen concentration and the measured value are plotted.
      On the other hand, when a substrate which emits light is used, detection can be conducted by radioautography using an X-ray film or an imaging plate or photography using an instant camera in a Western blot technique or a dot/slot blot method, and quantification by densitometry or BAS2000II system is also possible. Moreover, when using a light-emitting substrate in the ELISA method, enzyme activity is measured using light-emission micro plate readers (for example, manufactured by Bio-Rad or the like).
   5) Measurement operation
      i) In the case of Western blotting, a dot blot method, or a slot blot method
         First, in order to prevent nonspecific adsorption of an antibody, a membrane is immersed in a buffer solution containing a substance which inhibits such a nonspecific adsorption (skimmed milk, bovine serum albumin, gelatin, polyvinylpyrrolidone, or the like) for a certain time (blocking) in advance. As the blocking solution, for example, phosphate buffered saline (PBS) or Tris buffered saline (TBS) containing 5 % of skimmed milk and 0.05 to 0.1 % of Tween 20 is used. Instead of skimmed milk, 1 to 10% of bovine serum albumin, 0.5 to 3% of gelatin, or 1% of polyvinylpyrrolidone or the like can be used. The blocking time is 16 to 24 hours at 4 °C, or 1 to 3 hours at a room temperature.
         Then, after washing a membrane with PBS or TBS containing 0.05 to 0.1% of Tween 20 (hereinafter referred to as "washing solution") to remove any excess blocking solution, the antibody produced by the method of the above 3) is dipped for a certain time in the solution diluted suitably with the washing solution to allow the antibody to bind with the antigen on the membrane. The dilution rate of the antibody at this time can be determined by conducting a preliminary Western-blotting experiment using the stepwise diluted recombinant antigen described in the above 3) as a sample. This antibody reaction operation is preferably performed at room temperature for one hour. The membrane is washed with a washing solution after completion of the antibody reaction operation. When a labeled antibody is used, the detection operation can be performed immediately at this time. When a non-labeled antibody is used, a second-antibody reaction is performed thereafter. The labeled second antibody is diluted 2000 or 20000 times by the washing solution, in the case that it should be commercial (if a suitable dilution rate is indicated in an attached direction, it should be diluted in accordance with it). The membrane from which the primary antibody is removed by washing is dipped in a solution of the secondary antibody at room temperature for one to 3 hours, and detection corresponding to a labeling method is conducted following washing with the washing solution. The washing is conducted by incubating the membrane in the washing solution for 15 minutes, renewing the washing solution, incubating it for 5 minutes, and renewing the washing solution again, and then incubating it for 5 minutes. If necessary, the washing solution is then renewed and further washing takes place.
      ii) ELISA/RIA
         First, in order to prevent nonspecific adsorption of the antibody to the bottom of a well of a plate on which the sample is immobilized by the method of the above 2), blocking is conducted in advance as in the case of Western blotting. The conditions of blocking are described in the paragraph concerning Western blotting.
         Then, after washing the inside of the well with PBS or TBS containing 0.05 to 0.1 % of Tween 20 (hereinafter referred to as "washing solution") to remove any excess blocking solution, a solution obtained by suitably diluting the antibody produced by the method of the above 3) with the washing solution is poured distributively, and incubated for a certain time, and thereby the antibody is bound to the antigen. The dilution rate of the antibody at this time can be determined by conducting a preliminary ELISA experiment using a recombinant antigen of the above 3) which is stepwise diluted as a sample. This antibody reaction operation is preferably performed at room temperature for about 1 hour. The membrane is washed with the washing solution after completion of an antibody reaction operation. When the antibody is labeled, the detection operation can be performed immediately at this time. When the antibody is not labeled, a second-antibody reaction is performed thereafter. The labeled second antibody should be diluted 2000 or 20000 times by the washing solution, in the case that it is commercial (if a suitable dilution rate is indicated in an attached direction, it should be diluted in accordance with it). The membrane from which the primary antibody is removed by washing is immersed in a solution of the secondary antibody at room temperature for 1 to 3 hours, and detection corresponding to a labeling method is conducted following washing with the washing solution. The washing is conducted by incubating the membrane in the washing solution for 15 minutes, renewing the washing solution, incubating it for 5 minutes, and renewing the washing solution again, and then incubating it for 5 minutes. If necessary, the washing solution is then renewed and further washing takes place.
         In the present invention, a so-called sandwich ELISA can be carried out by the method indicated below. First, in any one of the amino acid sequence shown in the amino acid number 17-455 (preferably 19-455) of SEQ ID No. 2 of the Sequence Listing, and the amino acid sequence shown in the amino acid number 17-460 of SEQ ID No. 4, two highly hydrophilic domains are chosen, and partial peptides consisting of six or more amino acid residues in each domain are synthesized, and then two kinds of antibodies for these partial peptides as an antigen are obtained. One of these antibodies is labeled as described in the above 4). The antibody which is not labeled is immobilized on the bottom of a well of the 96 well plate for ELISA according to the method described in the above 2). After blocking, the sample liquid is put in the well and incubated at ordinary temperature for one hour. After washing the inside of the well, the diluent of the labeled antibody is poured distributively to each well. After washing the inside of the well again, a detection operation corresponding to the labeling method is conducted.
   6) Evaluation
      The detection results obtained by the method described above are compared between the sample originating from the cell cultured in the presence of the test substance, and the sample originating from the cell cultured in the absence of the test substance. As a result, the test substance for which the amount of production of the polypeptide to which the antibody binds specifically is reduced may be a therapeutic or preventive agent for hyperlipidemia. Moreover, a kit for examining a therapeutic or preventive agent for hyperlipidemia can be provided by packing the antibody produced by the method described in the above 3), and the reagents used for a series of the above-mentioned methods.

The polypeptide to be detected in the method of the present invention can be obtained according to the above-mentioned method of obtaining the antigen for preparation of the antibody production, or obtained by making animal cells to produce it using adenovirus vector in which DNA having the nucleotide sequence shown in the nucleotide number 47-1411 of SEQ ID No. 1 of the Sequence Listing, or the nucleotide sequence shown in the nucleotide number 78-1457 of SEQ ID No. 3 of the Sequence Listing is incorporated. The method of constructing such a recombinant adenovirus vector can be a method using a commercial kit (for example, an adenovirus expression vector kit, TAKARA SHUZO CO., LTD.). The fact that the gene to be detected in the method of the present invention correlates closely with the neutral-fat concentration in the blood of mammals can be proved by the fact that elevation of neutral-fat concentration in blood is observed when a mammal, for example, a mouse is injected with a recombinant adenovirus having the recombinant adenovirus vector obtained as mentioned above, and the gene carried by the recombinant adenovirus vector is then expressed, and by the fact that the degree of expression of the nucleotide sequence shown in the nucleotide number 47-1411 of SEQ ID No. 1 of the Sequence Listing in a congenitally hypolipidemic mouse is lower than in a hyperlipidemic mouse.

Moreover, the present invention also relates to a method for testing a therapeutic or preventive agent for hyperlipidemia using a non-human animal to which a foreign gene containing the nucleotide sequence described in any one of the above a) to e) by genetic manipulation so that the gene can be expressed highly. The animal used for the method may be, for example, a KK/San mouse with which the above-mentioned recombinant adenovirus has been infected, or a transgenic mouse obtained by introducing into the mouse DNA having the nucleotide sequence shown in the nucleotide sequence shown in the nucleotide number 47-1411 of SEQ ID No. 1 of the Sequence Listing or the nucleotide number 78-1457 of SEQ ID No. 3 of the Sequence Listing, but it is not limited thereto.

A transgenic animal is obtained by taking the fertilized ovum from an animal, introducing the gene into it, and transplanting it to a false-pregnancy animal and generating it after transgenics. An already-established method can be followed [See "Hasseikougaku mannual" , edited by Tatsuji Nomura and Motonari Katsuki, 1987 annual publications), "Manipulating the Mouse Embryo and A Laboratory Manual" B. Hogan, F. Costantini and E. Lacy, translated by Kazuya Yamauchi, Hiroshi Toyoda, Hiroatu Mori, Yoichiro Iwakura, 1989, and Japanese patent publication (Kokoku) No.5-48093]. Specifically, in the case of a mouse, for example, an ovulation inducing agent is administrated to a female mouse (a mouse in which the neutral-fat concentration in the blood is lower than usual is preferable, for example, a KK/San mouse is preferable, but not limited thereto), and crossed with a male of the same line, and then a pronucleus fertilized ovum is extracted from the oviduct of the female mouse on the next day. Subsequently, a DNA fragment solution is introduced into the pronucleus of the fertilized ovum using a minute glass tube. Any regulatory genes for expressing the gene in the animal cell such as a promoter and an enhancer can be used, as long as they function in the cell of the introduced animal. The fertilized ovum to which DNA is introduced is transplanted to an oviduct of a false pregnancy female mice as an adoptive mother (Slc:ICR or the like), and is delivered by natural birth or by cesarean section after about 20 days. A method of checking whether the thus-obtained animal has the introduced gene may be a method of extracting DNA from the tail of this animal or the like and performing PCR using a specific sense primer and antisense primer to the introduced gene and using the above DNA as a template, a method of digesting this DNA with several sorts of restriction enzymes followed by electrophoresis and blotting of the DNA on the gel of a nitrocellulose membrane, a nylon film, or the like, and then performing a Southern blotting analysis using all or a part of the labeled gene which is the same as the introduced gene as a probe. Moreover, it can be checked whether the introduced gene is actually expressed in the animal body by measuring the neutral-fat concentration in the peripheral blood. When the introduced gene is actually expressed in the animal's body, the neutral-fat concentration in the blood becomes higher than an animal into which the gene is not introduced.

The test substance is administrated to the transgenic animal thus obtained, and the substance which reduces neutral-fat concentration in blood is chosen (preferably, the test substance is also administrated to the animal to which the gene is not introduced, and the results are compared, and the substance which notably reduces the neutral-fat concentration in blood in the transgenic animal is chosen). According to this method, not only substances that suppress or inhibit expression of the introduced gene but also substances which inhibit any of the vital reactions connected to the elevation of the neutral-fat concentration in blood by an action of a polypeptide, such as a factor contributing to expression of a function of the polypeptide itself or a function of interacting with this polypeptide to express the function of the polypeptide (for example, a receptor), can be found out. Such a substance may also serve as a therapeutic or preventive agent for hyperlipidemia.

The present invention relates to a polypeptide other than an antibody which specifically binds to the polypeptide to be detected by the method of the present invention (hereinafter referred to as "polypeptide to be detected"), namely a receptor. When the receptor is a protein which exists in a cell membrane, the receptor can be cloned according to the following method. First, a recombinant vector by which DNA consisting of a nucleotide sequence shown in the nucleotide numbers 47-1411 of SEQ ID No. 1 of the Sequence Listing or the nucleotide sequence shown in the nucleotide numbers 78-1457 of SEQ ID No. 3 of the Sequence Listing can be expressed in a mammalian cell is constructed, then introduced into COS-1, and culture supernatants are collected followed by purification of the recombinant polypeptide of the polypeptide to be detected. The obtained recombinant polypeptide is labeled with fluorescein isothiocyanate (hereinafter referred to as "FITC"). Then, the labeled recombinant polypeptide is added to cultured cells originating from various mammalian origins, and the cells wherein the labeled recombinant polypeptide specifically binds to a cell membrane thereof, namely the cells which express a receptor are identified. The cDNA library originating from the identified cells is incorporated into vectors which can be expressed in a mammalian cell, and allowed to be expressed in cells which do not express a receptor (preferably COS- 1 or a CHO cell, more preferably a CHO cell). The recombinant polypeptide labeled with FITC is added to the cells where the cDNA library is expressed, and the cells are collected after culturing for a certain time, and then the cells to which the recombinant polypeptide labeled with FITC binds are selected by a cell sorter. The introduced cDNA is cloned from the obtained cells by PCR or the like. If necessary, the above operation is repeated, and finally, the cDNA encoding the receptor specifically binding to the FITC-labeled recombinant polypeptide is cloned. Moreover, the receptor itself is recoverable from the cell cloned as above.

Thus, if cDNA which encodes the receptor of the polypeptide to be detected is obtained, the cells used as the material for the cDNA library are further used for screening of substances which inhibit the interaction between the polypeptide to be detected and the receptor, and the substances which bind to the receptor and have the same function as the polypeptide to be detected or the substances which inhibit the signal transfer initiated by the interaction between the polypeptide to be detected and the receptor. Specifically, the mouse genomic DNA is suitably fragmented with a restriction enzyme digestion or the like, and a vector wherein DNA encoding green-fluorescent protein is connected to the end thereof (pEGFP-1 (manufactured by Clontech ) is marketed as a vector for reporter assay) and this is introduced into the cells used as the material of the above-mentioned cDNA library. During culturing of the cells, the recombinant polypeptide (which is not labeled) of the polypeptide to be detected is added, and the cells which produce green-fluorescent protein are sorted out by the cell sorter. The cells sorted out produce green-fluorescent protein in the presence of this recombinant polypeptide. The cells are cultured so that the number of cells per well may become almost the same in each well of a 96 well culturing plate, and after culturing for a certain period after addition of only the test substance, or after addition of the recombinant polypeptide and the test substance simultaneously, the production amount of the green-fluorescent protein is measured using a fluorescence plate reader or the like. If production of green-fluorescent protein is caused when culturing only with the test substance, the substance is considered to be an agonist of the polypeptide to be detected. On the other hand, when the production amount of the green-fluorescent protein in the well to which the recombinant polypeptide and the test substance are simultaneously added is lower than the amount thereof in the well to which only the recombinant polypeptide is added, this substance is an antagonist of the polypeptide to be detected, or the signal transfer repressor of this polypeptide, and is considered to be useful as a therapeutic or preventive agent for hyperlipidemia.

When the antagonist thus obtained is a protein or a peptide, the polynucleotide which has the nucleotide sequence encoding the protein or the peptide can be used for gene therapy of hyperlipidemia. Such a polynucleotide can be obtained, for example, by analyzing the amino acid sequence of the identified antagonist protein or the polypeptide, and synthesizing an oligonucleotide probe which consists of a nucleotide sequence encoding the amino acid sequence, and performing screening of various cDNA libraries or genomic libraries. Moreover, when the peptide having an antagonist activity originates from an artificial peptide library synthesized at random, DNA consisting of a nucleotide sequence encoding the amino acid sequence of the peptide is chemically synthesized. In gene therapy, the polynucleotide which encodes the antagonist obtained as above is incorporated, for example, into a virus vector, and a patient is infected with the virus (detoxicated) which has the recombinant virus vector. In the patient's body, an antagonist is produced, and the function of the polypeptide which consists of an amino acid sequence shown in SEQ ID No. 4 of the Sequence Listing is inhibited, and therefore the neutral-fat concentration in the blood can be reduced.

As a method of introducing a gene therapy agent in a cell, either the transgenics method using the virus vector or the non-viral transgenics method (Nikkei science, April, 1994, 20-45 pages, an experimental-medicine special number, 12 (15) and (1994), an experimental-medicine separate volume "the basic technology of gene therapy", Youdosha (1996)) is applicable.

Examples of a method of introducing a gene into a cell with a virus vector include a method of incorporating DNA which encodes TR4 or variant TR4 into a DNA virus or RNA virus such as a retrovirus, adenovirus, an adenovirus related virus, Herpesvirus, vaccinia virus, a poxvirus, poliomyelitis virus, and Sindbis virus. Among them, methods using a retrovirus, adenovirus, an adenovirus related virus, and the vaccinia virus are especially preferable. As the non-viral transgenics method, the method of administering an expression plasmid to muscles directly (DNA vaccine method), a liposome method, a lipofectin method, a microinjection, a calcium phosphate method, an electroporation method or the like are mentioned, and the DNA vaccine method and the liposome method are especially preferable.

Moreover, in order to make a gene therapy agent act as a therapeutic agent, there are an in vivo method which introduces DNA into the body directly and an ex vivo method wherein a certain kind of cell is taken out from a human, DNA is introduced into the cell outside a body, and the cell is then returned into the body (Nikkei science, April 1994, 20-45 pages, Gekkanyakuji, 36 (1), 23-48 (1994), Jikkenigaku zoukan, 12 (15), (1994)).

For example, when the gene therapy agent is administered to the patient by the in vivo method, the medicine is administrated to the patient by a suitable administration pathway, such as intravenous, intra-arterial, hypodermical, intradermical, and intramuscular depending on the disorder, the symptom, or the like. Moreover, although this gene therapy agent is generally formulated as an injection or the like when it is administered to the patient by the in vivo method, a conventionally-used carrier may be added if necessary. Moreover, when it is formulated in the form of a liposome or a membrane-fusion liposome (Sendai-virus liposome or the like), it can be formulated as a liposome preparation, such as a suspension, cryogen, or centrifugal-separation concentrated cryogen or the like.

A nucleotide sequence complementary to the partial sequence of the nucleotide sequence shown in the nucleotide number 78-1457 of SEQ ID No. 3 of the Sequence Listing can be used for a so-called antisense treatment. An antisense molecule may be used as DNA which usually consists of a 15 or 30 mer complementary to a part of the nucleotide sequence shown in the nucleotide number 78-1457 of SEQ ID No. 3 of the Sequence Listing, or a stable DNA derivative such as a phosphorothioate, methyl phosphonate, or a morpholine derivative or the like, or a stable RNA derivative such as 2'-O-alkyl RNA. Such an antisense molecule can be introduced into a cell by a method well known in the technical field of the present invention, such as microinjection, liposome capsulation, or expression using a vector having an antisense sequence or the like. Such an antisense therapy is useful for a disease wherein it is useful to reduce the activity of the protein encoded by the nucleotide sequence shown in the nucleotide number 78-1457 of SEQ ID No. 3 of the Sequence Listing, especially for treatment of hyperlipidemia.

A composition useful as a medicine containing the above-mentioned antisense oligonucleotide may be manufactured by well known methods, such as mixing with a carrier permissible as a medicine. Examples of such carriers and manufacture methods are described in Pharmaceutical Sciences by Remington. A sufficient amount of the medicine for the treatment of the hyperlipidemia wherein expression of the gene containing the nucleotide sequence shown in the nucleotide number 78-1457 of SEQ ID No. 3 or the activity of the gene product is abnormal is administrated to each of them. The effective dose may be varied due to various factors such as conditions, weight, sex, and age, and due to difference in the administration method such as hypodermical, local, oral and intramuscular. For example, it is 0.02 to 0.2 mg/kg/hour for 2 hours when administrated by an intravenous injection, and 1 to 200 mg/m²/day in the case of hypodermical administration.

### [Brief explanation of the drawings]

Fig. 1 is a view showing the result of a Northern blotting analysis of samples originating from the organs of a KK mouse. The numbers on the right-hand side of the lanes (28S and 18S) show the sedimentation coefficients of marker RNAs.
Fig. 2 is a view showing the result of the Western-blotting analysis using a transfected COS-1 cell culture supernatant as a sample. The numbers on the left-hand side of the lanes show the molecular weights (KDa) of molecular weight markers.
Fig. 3 is a view showing the result of the Western-blotting analysis using a transfected HeLa-cells culture supernatant as a sample. The numbers on the left-hand side of the lanes show the molecular weight (KDa) of molecular weight markers.
Fig. 4 is a view showing results of measurement of neutral-fat concentration in the peripheral blood of a KK/San mouse infected with a recombinant adenovirus.
Fig. 5 is a view showing the result of the Northern blotting analysis for a sample originating from the liver of a KK mouse and a KK/San mouse infected with a recombinant adenovirus. The numbers on the left-hand side of the lanes show the sedimentation coefficients of marker RNAs.

### [Best Mode of Carrying Out the Invention]

The present invention will be explained below in more detail with reference to examples, but the present invention is not limited thereto. In addition, in the following examples, each operation by genetic manipulation was conducted according to the method described in 1989 "Molecular Cloning" [Sambrook, J., Fritsch, E.F. and Maniatis, T., Cold Spring Harbor Laboratory Press], unless otherwise indicated. Alternatively, if a commercial reagent and a commercial kit were used, it is conducted according to directions therein.

### Reference example 1. Cloning of cDNA

The cDNA having the nucleotide sequence shown in SEQ ID No. 1 of the Sequence Listing was obtained using mouse liver as a material according to the following method.
a) Extraction of mRNA from mouse liver
   Two 9 week old KK mice (male, obtained from the Animal experiment institution attached to Hamamatsu University School of Medicine) were dissected, and their livers were extracted and put into liquid nitrogen promptly for quick freezing. The weight was measured and 3.1 g of it were ground in a mortar in the presence of liquid nitrogen. Thereto was added 5.5 M guanidine thiocyanate buffer solution (hereinafter referred to as GT) (5.5 M guanidine thiocyanate, 25 mM sodium citrate (pH 7.0), 0.5% sarkosyl, 0.2 M β-mercaptoethanol) (30 ml). Then, it was crushed with a pestle, then GT buffer solution (10 ml) was newly added, and crushed with the pestle, and the solution was recovered. Then, the mortar was washed with GT buffer solution (20 ml) and the solution was also recovered. After 36 ml of the recovered solution were centrifuged at 3000 rpm, at 10 °C for 10 minute, the supernatant was transferred to a new tube, and suction and eccrisis were repeated 20 times with an 18 gauge injection needle. Then, total RNA was separated by density-gradient centrifugation using cesium trifluoroacetic acid (CsTFA). CsTFA stock solution (19 ml) was diluted with ribonuclease-free distilled water (18.924 ml), and the diluent (6.18 ml) was put into six 13PA tubes (manufactured by Beckmann), and the sample (5.2 ml per one tube) collected previously was layered thereon. After carrying out centrifugation at 30000 rpm (about 125000 g), at 20 °C with an ultracentrifuge (Hitachi SCP70H type) for 20.5 hours using a swing rotor (Hitachi Koki CO., LTD. P40 ST), the pellet obtained by removing the supernatant was suspended in 3.3 ml of a buffer solution for extraction appended to an mRNA purifying kit (Quick prep mRNA purifying kit manufactured by Amersham Pharmacia). The mRNA was purified using the purifying kit according to the appended protocol. A lambda-phage cDNA library was produced using 5 µg of mRNA thus obtained as a template with a cDNA library production kit (ZAP express, cDNA Giga pack III gold cloning kit manufactured by Stratagene) according to the appended protocol.
b) Primary screening of a cDNA library
   Escherichia coli infected with the lambda-phage cDNA library obtained by the above-mentioned method was dispersed on an agar plate (NZY culture medium: 0.5 % sodium chloride, 0.2 % magnesium sulfate heptahydrate, 0.5 % yeast extract, 1 % casein hydrolysate and 1.5 % agar) prepared in a culture laboratory dish with a diameter of 9 cm so that 1.8 x 10⁵ plaques per plate may be formed, and cultured at 37 °C for 8 hours. At 14 places of this agar on which the plaque was formed, the agar including the plaque was sampled using the bottom part of a large diameter 250 µl pipette (manufactured by RAININ), and the pieces of these agar were respectively put into a plastic centrifugation vessel containing 100 µl of SM buffer solution (0.1 M of sodium chloride, 8 mM of magnesium sulfate, 50 mM of Tris- hydrochloric acid (pH 7.5), and 0.01 % of gelatin), agitated using a vortex mixer to make it cloudy and then left at 4 °C for 1 to 2 hours. Then, the supernatants were recovered by centrifugation at 12000 x g for 5 minutes, and used as a phage suspension.
   As a primer used for PCR, oligonucleotides having the following nucleotide sequences were synthesized using an automatic DNA-synthesis machine (model 394 : product manufactured by Perkin-Elmer Japan Applied biotechnology systems operation division) according to a phosphoramidite method (Matteucci, M.D., and Caruthers, M. H. (1981) J. Am. Chem. Soc. 103, 3185-3191).
   Primer 1: 5'-gactgatcaa atatgttgag ctt -3' (SEQ ID No. 5 of the Sequence Listing);
   Primer 2: 5'-tgcatccaga gtggatccag a -3' (SEQ ID No. 6 of the Sequence Listing)
   5 µl of the thus obtained phage suspension were mixed with 2.5 µl of a buffer solution for 10 x PCR (appended to Taq polymerase by TAKARA SHUZO CO., LTD.), 4 µl of a dNTP mixture (2.5 mM each, appended to Taq polymerase by TAKARA SHUZO CO., LTD.), 1 µl each of the above-mentioned primers 1 and 2 adjusted to 7.5 µM, 0.25 µl of Taq polymerase (TAKARA SHUZO CO., LTD.) and 11.25 µl of sterilized water, and then the mixture was heated first at 94 °C for 5 minutes, then there was repeated 30 times a cycle of for 30 seconds at 94 °C, for 30 seconds at 55 °C, and for 30 seconds at 72 °C, then the mixture was finally kept for 7 minutes at 72 °C, and then it was stored at 4 °C. The reactant was subjected to electrophoresis on a 4 % agarose gel (NuSieve 3:1 agarose (manufactured by FMC bioProducts)), and analyzed for amplification of a specific fragment. As a result of the above screening of 14 phage suspensions, two positive samples in which the intended cDNA fragment was amplified were obtained.
c) Secondary screening
   The DNA fragments amplified by performing PCR under the same conditions as described in the above b) using 100 ng of a mouse genomic DNA (manufactured by Clontech) as a template were collected by performing agarose electrophoresis. DNA fragments labeled with ³²P were produced with a multi-prime DNA labeling system (manufactured by Amersham Pharmacia) using these DNA fragments as templates, and the reaction mixture was poured into a nick column (Amersham Pharmacia). 400 µl of TE (10 mM Tris-hydrochloric acid (pH 7.5), 1 mM EDTA) were passed through the column once for washing, and a further 400 µl of TE were passed through, and the eluates were collected. All of the eluted fractions were used for the following secondary screening as a labeled probe.

On the other hand, the phage suspension judged to be positive by the above b) was diluted with SM buffer solution 100 times, the Escherichia coli infected with 2 µl of the phage was distributed on an agar plate prepared on the laboratory culture dish with a diameter of 9 cm and cultured at 37 °C for 8 hours. On this agar on which the plaques were formed, a circular nylon membrane (manufactured by Amersham Pharmacia, High bond N⁺) fitted to the inside diameter of the laboratory dish was placed, and the plaques were transferred by leaving it for 5 minutes at 4 °C. At three places, the membrane was penetrated to the agar using an 18G injection needle to put a mark for positioning, and then the membrane was removed, dipped in an alkali solution (1.5 M sodium chloride, 0.5 M sodium hydroxide) for 2 minutes, then in a neutralization solution (1.5 M sodium chloride, 0.5 M Tris- hydrochloric acid (pH 8.0)) for 5 minutes, and further in a solution containing 2 x SSC and 0.2 M Tris-hydrochloric acid (pH 7.5) for 30 seconds, and subsequently air-dried completely at room temperature.

After incubating (pre-hybridization) the membrane in 20 ml of a hybridization solution (Express Hyb Hybridization Solution, manufactured by Clontech) at 68 °C for 1 hour, the solution was replaced by 8 ml of a hybridization solution containing a labeled probe, and incubated at 68 °C for 6 hours. Then, the operation was conducted three times of washing the membrane with a solution containing 2 x SSC and 0.05 % of SDS at a room temperature for 15 minutes, then the operation was conducted three times of washing it with a solution containing 2 x SSC and 0.05% of SDS with shaking gently for 15 minutes, and further the operation was conducted 3 times of washing it with a solution containing 0.1 x SSC, 0.1 % of SDS at 50 °C for 30 minutes.

The membrane after washing was subjected to radioautography, and the original plaques at the position accepted to be a positivity were collected from the agar, and the phage suspension subjected to PCR under the same conditions indicated in the above b). Specific amplification of the DNA fragment was recognized in six pieces among ten positive plaque samples.

The phage suspension of the sample wherein amplification was the most strong among these, was subjected to in vivo excision using a helper phage and a host bacterium which were appended to ZAP express cDNA Giga pack III gold cloning kit (manufactured by Stratagene) according to the protocol appended to the kit, to make Escherichia coli colonies which contain a phagemid on the agar. These colonies were isolated, and the phagemids were extracted respectively, and subjected to PCR by the method described in the above b). As a result, the colonies where specific amplification of the DNA fragment was recognized was chosen and cultured, to isolate transformed Escherichia coli which carries phagemid #55-1 having the cDNA insert of 1.6 kbp, E.coli pBK/m55-1 SANK72199.

All of the nucleotide sequence of the cDNA incorporated in phagemid #55-1 thus obtained was analyzed with an ABI prism 377 DNA sequencer manufactured by Perkin-Elmer Japan biotechnology systems operation division, or by a 3700 DNA sequencer. As a result, it was revealed that it is the sequence shown in SEQ ID No. 1 of the Sequence Listing (however, the nucleotide number 1-8 of SEQ ID No.1 of the Sequence Listing is the adapter sequence originating from the vector). This sequence was the same as that of the sequence (registration number : AF162224) registered into the GenBank database as a mouse angiopoietin related protein 3. In addition, transformed Escherichia coli, E.coli pBK/m55-1 SANK72199 carrying the phagemid #55-1 was internationally deposited on November 19, 1999 with the Kogyo Gijutsuin Seimei-Kogaku Kogyo Gijutsu Kenkyujo (National Institute of Advanced Industrial Science and Technology, International Patent Depositary) of the Japan 1-1-3, Higashi, Tsukuba-shi, Ibaraki-ken, and was accorded the accession number FERM BP-6940.

### Example 1. Northern blotting analysis

a) Extraction of total RNA from mouse organs
   Northern blotting analysis was carried out in order to determine the organ where cDNA obtained in the Reference Example 1 was expressed. First, total RNA was extracted from the testis, spleen, kidney, small intestine, liver, and brain of KK mouse (hyperlipidemic mouse) and a KK/San mouse (hypolipidemic variation mouse, Shiraki et al., the 7th diabetes animal study group (1993)). The 18 week old KK mouse and the KK/San mouse were dissected, then each of the organs thereof was extracted, and then put in liquid nitrogen promptly to be cooled rapidly, and stored at -80 °C. About 15 ml of TRIzol reagent (product manufactured by Gibco BRL) were added to 0.5 g each of the organs, and homogenized on ice using an ultra high-speed homogenizer Polytoron (manufactured by Ckinematica) (graduation 6, for 2 minutes). After leaving it for 5 minutes at room temperature, 3 ml chloroform were added thereto and it was vigorously mixed by hand for 15 seconds. After leaving it for 3 minutes at room temperature again, it was centrifuged at 12000 x g and at 4 °C for 15 minutes. The upper layer was collected after centrifugation, and 0.8 volume of ribonuclease-free isopropyl alcohol was added and mixed. After leaving it at room temperature for 10 minutes and then centrifuging it at 12000 x g at 4 °C for 10 minutes, the supernatant was removed and ribonuclease-free 70% ethanol was added thereto. After centrifugation at 12000 x g at 4 °C for 10 minutes, the supernatant was removed and the precipitate was dried and then stored at -80 °C.
b) Electrophoresis and blotting of total RNA
   The total RNA of each collected organ was prepared to be 4 µg/µl with ribonuclease-free distilled water, and then 5 µl of the RNA solution and 16 µl of a RNA sample buffer solution (1.15 x MOPS buffer solution (1 x MOPS buffer solution contains 20 mM of MOPS, 5 mM of sodium acetate and 1 mM of ethylenediamine tetraacetic acid (hereinafter referred to as "EDTA")), 2.4 M of formaldehyde, 57 % of formamide, 7 % of glycerol, 18 µg/ml of Bromophenol Blue, 18 µg/ml of xylene cyanol, and 0.18 mM of EDTA) were mixed, kept at 65 °C for 10 minutes, and then left on ice for 5 minutes. The whole amount of this sample solution was poured into one well containing the agarose gel for electrophoresis (1 x MOPS buffer solution, 1.17 % agarose (high strength, for analysis, manufactured by Bio-Rad), 0.66 M formaldehyde) containing 1.17 % formalin, and subjected to electrophoresis. The electrophoresis was performed at 50 V for about 1 hour, and then at 100 V for about 1.5 hours, in the submarine electrophoresis tub containing 1 x MOPS buffer solution containing 500 ng/ml ethidium bromide. After the electrophoresis, the RNA in the agarose gel was transferred to a nylon membrane (High bond N+, manufactured by Amersham Pharmacia) overnight (20 x SSC was used as the solution for transferring) according to the capillary transfer method (Maniatis, T. et al. (1982) in "Molecular Cloning A Laboratory Manual" Cold Spring Harbor Laboratory, NY). The membrane was washed with 2 x SSC for 5 minutes and air dried, and then inrradiated with ultraviolet rays using an ultraviolet ray irradiating apparatus to give crosslinking (Spectrolinker XL-1000, Tomy seiko) (1200 J/cm²), and thereby the RNA was fixed.
c) Preparation of a probe
   PCR was performed under the following condition using a Thermal cycler (Gene amplifier PCR system 9600, manufactured by Perkin-Elmer Japan Applied biotechnology systems operation division) using the primer synthesized in b) of Reference Example 1. After adding sterilized water to the primer (final-concentration 0.5 µM each) and Tween 20 (manufactured by Sigma, final concentration of 0.1 %) to give 7.5 µl, 7.5 µl of 2 x PCR solution premix Taq (manufactured by TAKARA SHUZO CO., LTD.: 0.05 unit/µl Taq polymerase, 0.4 mM dNTPs, 20 mM Tris-hydrochloric acid (pH 8.3), 100 mM potassium chloride, and 3 mM magnesium chloride) were added. Furthermore, 1 µl (an equivalent to 100 ng) of mouse genomic DNA (manufactured by Clontech) was added thereto, and thereby the reaction mixture was prepared. After heating the reaction mixture for 3 minutes at 94 °C first, a cycle of heating at 94 °C for 30 seconds, at 55 °C for 1 minute and at 72 °C for 45 seconds was repeated 35 times, and then the mixture was kept at 4 °C.
   One µl of the reaction mixture after PCR was taken, and the amplified DNA fragment was cloned into a plasmid vector using a TA cloning kit (Dual promoter version A, manufactured by Invitrogen) according to the attached protocol. A competent strain of Escherichia coli was transformed with the recombinant plasmid vector, and cultured on LB agar containing 50 µg/ml ampicillin. The Escherichia coli colonies showing ampicillin resistance that grew as a result were chosen, and cultured at 37 °C overnight in 4 ml of liquid LB culture medium containing 50 µg/ml of ampicillin. From this, plasmid DNA was collected from 3.5 ml of liquid medium using a plasmid automatic extractor (PI-50, manufactured by Kurabo Industries, Ltd.). The nucleotide-sequence of the obtained plasmid DNA was analyzed, and the plasmid in which the target PCR product was incorporated was used for the following operations.
   After digesting 8 µg of the selected plasmid DNA with the restriction enzyme EcoRI, phenol/chloroform extraction and ethanol precipitation were performed. The obtained precipitate was dissolved in 10 µl of sterilized water. To the solution was added 2 µl of a pigment solution (0.25% Bromophenol Blue, 0.25% xylene cyanol, 15% Ficoll (Type 400)), and then subjected to polyacrylamide gel electrophoresis (8% gel concentration, 100V, at room temperature, for 3 hours). After the electrophoresis, the gel was dyed with ethidium bromide, the piece of the gel at the band equivalent to the target DNA (about 200 bp(s), SEQ ID No. 11 of the Sequencing Listing) under ultraviolet irradiation was cut out with a razor edge, and transferred to a microdose centrifugal tube and ground. Thereto was added 300 µl of an elution buffer solution (0.5 M ammonium acetate, 10 mM EDTA (pH 8.0), 0.1 % SDS), and this was kept at 37 °C overnight, and then phenol/chloroform extraction was performed twice, and ethanol the precipitate was performed once, and the precipitate was dissolved in 20 µl of sterilized water.
   Using 5 µl of the obtained DNA solution, a probe (400 µl) labeled with ³²P was prepared by the method of c) of Reference Example 1.
d) Hybridization
   After putting the membrane prepared in the above b) into 20 ml of hybridization solution (ExpressHyb Hybridization Solution, manufactured by Clontech) and carrying out an incubation at 68 °C for 1 hour (pre hybridization), incubation was carried out at 68 °C in 20 ml of a hybridization solution containing a ³²P labeled probe overnight. Then, the membrane was washed three times with a solution which contains 2 x SSC and 0.05% SDS at room temperature for 20 minutes and 3 times with a solution containing 0.1 x SSC and 0.1% SDS at 50 °C for 20 minutes, and thereafter radioautography was performed.

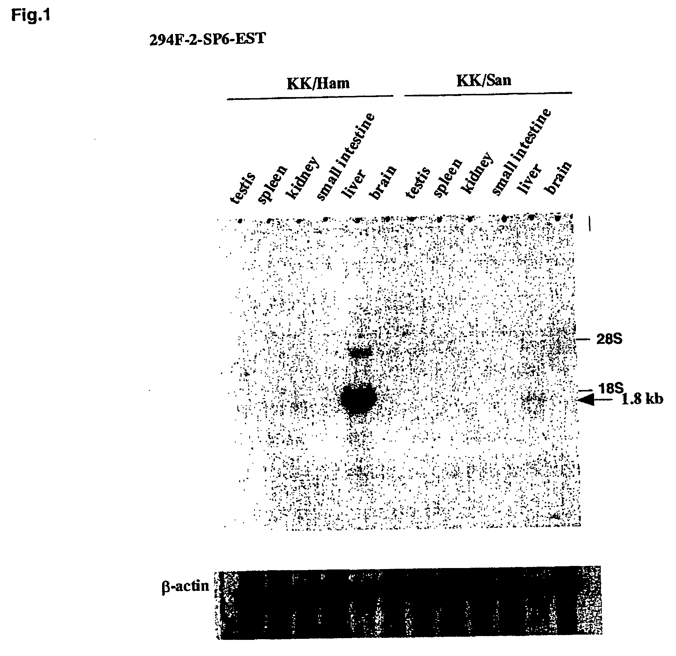

Consequently, expression of the detected gene was seen only in the liver, and it became clear that the expression level thereof was remarkably reduced in the KK/San mouse (hypolipidemic mouse) compared with in the KK mouse (hyperlipidemic mouse) (Fig. 1).

In the above-mentioned experimental system, the effect as a therapeutic or preventive agent for hyperlipidemia of the test substance can be investigated by preparing an RNA sample from primary-culture hepatocytes of KK mouse cultured in the presence or absence of the test substance, and performing the same operation as above. Test substances which reduce the expression level of the gene detected in this experiment may serve as a therapeutic or preventive agent for hyperlipidemia. When performing multi-test substance treatment, the electrophoresis can be omitted and a dot blot and a slot blot can also be performed.

### Reference example 2: Cloning of human cDNA

1) Preparation of a probe
   In order to acquire the human cDNA corresponding to the mouse cDNA shown in SEQ ID No. 1 of the Sequence Listing, oligonucleotide primers having the following nucleotide sequence were synthesized: 5'-tcctctagtt atttcctcca g -3' (SEQ ID No. 7 of the Sequencing Listing); and
   5'-tggtttgcca gcgatagatc -3' (SEQ ID No. 8 of the Sequence Listing).
   Then, one µl of human genome DNA (manufactured by Boeheringer Mannheim, 200 mg/ml), one µl of Taq polymerase (rTaq, TAKARA SHUZO CO., LTD., five units/µl), 10 µl of 10 x buffer solution for PCR (manufactured by TAKARA SHUZO CO., LTD.), 16 µl of dNTP mixed solution (2.5 mM each), 2 µl each of 20 µM primer, and 68 µl of sterilized water were mixed. The reaction mixture was heated at 94 °C for 5 minutes, and then a temperature cycle of heating at 94 °C for 30 seconds, at 55 °C for 30 seconds and at 72 °C for 30 seconds was repeated 30 times, and then it was finally heated at 72 °C for 10 minutes, and then stored at 4 °C. The reaction solution was subjected to electrophoresis on a 2 % agarose gel, and the gel at an amplified DNA band part was cut out and purified. The thus obtained DNA was labeled with ³²P using a DNA labeling kit (BcaBest labeling kit, TAKARA SHUZO CO., LTD.), and used as a probe in the following 2).
2) Primary screening of cDNA library
   1 x 10⁶ plaques of DNA from commercial cDNA libraries originating from human liver (Human Liver 5'-STRETCH cDNA Library, manufactured by Clontech) were fixed to a nylon membrane. Namely, Escherichia coli infected with the cDNA library was distributed in 20 agar plates which were created on laboratory culture dishes with a diameter of 9 cm so that 5 x 10⁴ plaques per sheet were formed, and then cultured at 37 °C for 8 hours. On the agar on which the plaque formation had been carried out, a circular nylon membrane (manufactured by Amersham Pharmacia, High bond N+) fitted to the inside diameter of the laboratory dish was placed thereon, and the plaques were transferred thereto by leaving it for 5 minutes at 4 °C. The membrane were penetrated at three places to the agar using an 18G injection needle to put a mark for positioning, and then the membrane was removed, dipped in an alkali solution (1.5 M sodium chloride, 0.5 M sodium hydroxide) for 2 minutes, then in a neutralization solution (1.5 M sodium chloride, 0.5 M Tris- hydrochloric acid (pH 8.0)) for 5 minutes, and future in a solution containing 2 x SSC and 0.2 M Tris-hydrochloric acid (pH 7.5) for 30 seconds, and subsequently air-dried completely at room temperature. Then, using a UV irradiation apparatus (Spectro-linker XL-1000, manufactured by Tomy seiko) (1200 J/cm²), the DNA was fixed.

The membrane thus prepared was incubated at 65 °C overnight in a hybridization solution (ExpressHyb Hybridization Solution, manufactured by Clontech). Then, the membrane was washed 3 times with a solution containing 2 x SSC and 0.05% of SDS with shaking gently for 15 minutes, and a further 3 times with a solution containing 0.1 x SSC, 0.1 % of SDS at 50 °C for 30 minutes, and then subjected to autoradiography.

The plaques which were at the position of the positive signal determined as a result were collected including the culture medium from the above-mentioned agar plate, and put into 100 µl of a SM buffer solution (0.1 M sodium chloride, 8 mM magnesium sulfate, 50 mM Tris- hydrochloric acid (pH 7.5), 0.01% gelatin) respectively, suspended therein and left at 4 °C for 2 hours. The supernatant was collected by centrifugation at 12000 x g for 5 minutes.

Escherichia coli infected with the thus obtained primary positivity phage liquid was cultured on agar medium produced on a laboratory culture dish with a diameter of 9 cm so that 500 plaques per laboratory dish were formed, and secondary screening was performed by repeating the above-mentioned operation. Escherichia coli strain BM25.8 (manufactured by Clontech) infected with the obtained secondary positive clone phage was cultured at 37 °C on the agar medium to form Escherichia coli colonies containing the phagemid. The colonies were isolated, cultured in a small amount of the liquid medium, and thereby phagemid was extracted. The insert was analyzed using restriction enzyme digestion, and Escherichia coli E.coli pTrip/h55-1 SANK72299 comprising a clone #h5-1 having a 1.6 kbp insert was isolated. The nucleotide sequence of the insert of this clone was analyzed, and it was confirmed that it was the same as the cDNA sequence registered in GenBank as human angiopoietin related protein 3 (registration number : AF152562) (SEQ ID No. 3 of the Sequence Listing. However, the nucleotide number 1-14 of SEQ ID No. 3 of the Sequence Listing is the adapter sequence originating from the vector.) In addition, the transformed Escherichia coli E.coli pTrip/h55-1 SANK72299 carrying the phagemid #h5-1 was internationally deposited on November 19, 1999 with the Kogyo Gijutsuin Seimei-Kogaku Kogyo Gijutsu Kenkyujo (National Institute of Advanced Industrial Science and Technology, International Patent Depositary) of the Japan 1-1-3, Higashi, Tsukuba-shi, Ibaraki-ken, and was accorded the accession number FERM BP-6941.

### Example 2. Production of a polyclonal antibody

The peptides having two kinds of amino acid sequences chosen from the domain conserved between the polypeptides of mice and humans as an antigen in order to produce an antibody which recognizes each polypeptide which has the amino acid sequence encoded by the nucleotide sequence shown in SEQ ID No. 1 and SEQ ID No. 3 of the Sequence Listing, namely the amino acid sequence shown in SEQ ID No. 2 of the Sequence Listing and SEQ ID No. 4 of the Sequence Listing: Glu-Pro-Lys-Ser-Arg-Phe-Ala-Met-Leu-Asp-Asp-Val-Lys-Cys (55-1-N1, SEQ ID No. 9 of the Sequence Listing) and
Cys-Gly-Glu-Asn-Asn-Leu-Asn-Gly-Lys-Tyr-Asn-Lys-Pro-Arg (55-1-C1, SEQ ID No. 10 of the Sequence Listing) were chemically synthesized (the used apparatus: the Perkin-Elmer Japan model 433). However, a cysteine residue is added to the C terminus of the original amino acid sequence in the amino acid sequence of 55-1-N1 to which a Keyhole limpet hemocyanin (hereinafter referred to as "KLH") as a carrier would bind later. On the other hand, the cysteine at the N terminus of 55-1-C1 to which KLH binds originates from the original amino acid sequence.

Then, 11.1 mg of the synthetic peptide 55-1-N1 and 21.5 mg of KLH, or 10.2 mg of 55-1-C1 and 21.2 mg of KLH were condensed using N-(6-maleimidecaproyloxy) succinimide (EMCS, manufactured by Dojinkagakukenkyusho) respectively (it was kept at room temperature for 15 hours using 0.02 M phosphoric-acid buffer solution (pH 7.5) which contains 8 M urea and 0.9 % sodium chloride as a condensation-reaction solvent). This reaction mixture was put into 8M urea solution, dialyzed to the running water and dialyzed to purified water, and then freeze-dried, to provide a KLH-bound peptide antigen. One ml of physiological saline was added to about 10 mg of these peptide antigens, and then converted into a fine suspension using an ultrasonic oscillation machine (sonicator), a vortex mixer, a glass rod or the like. Then, the whole amount was made up to 7.5 ml with a physiological salt solution, and one ml each thereof was subdivided to a vial, and then frozen and stored.

When immunizing, the antigen solution in one of the above-mentioned vials was dissolved, mixed with an equal amount of adjuvant, and then injected into the back of two rabbits hypodermically or intradermally, respectively. As the adjuvant, complete Freund's adjuvant was used. For the 2nd or later immunization, incomplete Freund's adjuvant was used. Immunization was performed 4 times every two weeks, after the 2nd immunization, test blood collecting was performed and the antibody titer in the serum was investigated by an enzyme immunoassay (ELISA), a solid phase method. Each antigen peptide was coated on a 96 well plate for ELISA (manufactured by Sumitomo Bakelite CO., LTD., 96 well H type), and a Western horseradish-peroxidase labeled anti-rabbit IgG antibody was used as a second antibody. The exsanguination was performed 13 days after the 4th immunization.

The antibody was purified after blood collecting using an affinity column. That is, the peptide (55-1-N1: 7.82 mg, 55-1-C1: 8.07 mg) was combined with the carrier EMC-agarose (about 5 ml) which was activated by reaction of N-(6-maleimidecaproyloxy succinimide (EMCS, manufactured by Dojinkagakukenkyusho) with an aminoalkyl agarose (manufactured by Bio-Rad, Affigel 102). The inactivation of the unreacted EMC group was carried out by treatment with 0.1 M hydrochloric acid mercaptoethylamine (5 mM EDTA being included). 85 ml of antiserum were doubly diluted with PBS (containing 0.02 M phosphoric acid buffer solution (pH 7.0), 0.9 % sodium chloride), a precipitate was obtained by the ammonium sulfate precipitation (final concentration 40 %) method, this precipitate was dissolved in PBS, and then dialyzed with PBS after desalting. The dialysis liquid was used as the rough IgG fraction. Chromatography operation with an affinity column was carried out in three steps. That is, 1/3 quantity of the rough IgG fraction was charged to the affinity column, and the operation of re-charging a bypassing fraction into the column was repeated 3 times. 40 ml of the combined solution of the bypassing fraction and the washing solution were collected as a non-adsorbing fraction. In order to remove antibody binding non-specifically to the column, it was washed enough with PBS containing 1M sodium chloride, and then 4 M magnesium chloride solution, 3.5 M potassium thiocyanate solution, and 0.1 M glycine hydrochloric acid buffer solution (pH 2.3) were poured in the column one by one, and the antibody specifically binding to the peptide fixed on the column carrier was eluted as an affinity purified antibody. Since the target antibody was contained in the eluate of 4 M magnesium chloride solution and 3.5 M potassium thiocyanate solution, each eluate dialyzed to PBS was used as an antibody in the following operations.

### Example 3. Expression and Western-blotting analysis in COS-1 cell

#h5-1 phagemid DNA obtained in Reference example 2 was digested with restriction enzymes EcoRI and XbaI, and subjected to 8 % polyacrylamide gel electrophoresis, and about 1.6 kb of the fragment containing cDNA was isolated and purified by the method described in c) of Example 1. At the same time, a high expression vector pME18S (Hara, T. et al. (1992) EMBO. J. 11, 1875-, edited by Takashi Yokota, Kennichi Arai, the biotechnology manual series 3, the gene-cloning experimental method, Yodosha, p18-20) was similarly digested with EcoRI and XbaI, and the ends were dephosphorylated, and ligated using the above-mentioned cDNA fragments and a DNA ligation kit (Version 2, TAKARA SHUZO CO., LTD.). Escherichia coli was transformed with the DNA, the resultant transformant was analyzed with the restriction enzyme of the plasmid DNA carried by the transformant. The strain having the 1.6kb DNA fragment was chosen, and designated as pMEh55-1.

Then, the transformed Escherichia coli carrying pMEh55-1 was cultured at 37 °C overnight in 100 ml of liquid LB culture medium containing 50 µg/ml ampicillin. From this medium, pMEh55-1 DNA was collected using a plasmid purifying kit (Wizard purefection plasmid DNA purifying system, manufactured by Promega), and purified by a cesium-chloride method.

COS-1 cells were transfected by the thus obtained plasmid pMEh55-1. The transfection of COS-1 cells was performed according to an electroporation method using transgenics equipment GTE-1 manufactured by Shimadzu Corp. That is, from the flask in which COS-1 cells were proliferated until they became semi-cofluent, the cells were recovered by trypsin EDTA treatment, and washed with PBS (-) buffer solution (TAKARA SHUZO CO., LTD.). Next, the cells were suspended at 6 x 10⁷ cells/ml in PBS (-) buffer solution. The plasmid DNA (pMEh55-1) collected by the above-mentioned method was diluted to 200 µg/ml with a PBS (-) buffer solution. 20 µl each of the cell suspension and the DNA solution were mixed, put into a chamber with an electrode interval of 2 mm, and pulses of 600V-30 µsec were given twice at intervals of 1 second. After cooling the chamber for 5 minutes at 4 °C, the cell-DNA mixture was added to 10 ml of DMEM which contained 10 % fetal bovine serum, transferred to a laboratory dish, and was cultured under 5 % CO₂ at 37 °C overnight. Then, the culture supernatant was removed and the cells were washed with serum free medium (DMEM), and then 10 ml of DMEM were added, and cultured for three days.

The culture supernatant was collected from the thus-obtained cell culture. 0.3 ml each of the serum-free culture supernatants of COS-1 cells transfected with negative control plasmid pME18S or pMEh55-1 were treated with trichloroacetic acid (hereinafter referred to as "TCA") to precipitate protein, and the precipitate was obtained by centrifugation. The precipitate was washed with acetone cooled with ice, air-dried, dissolved in a sample buffer solution (manufactured by Bio-Rad) containing 2-mercaptoethanol for SDS-polyacrylamide gel electrophoresis (SDS-PAGE). Then, SDS-PAGE was performed under reducing conditions using 4-20% polyacrylamide density-gradient gel (multi-gel 4/20, manufactured by Daiichikagaku).

After the electrophoresis, the band was transferred in a transcription buffer solution (192 mM glycine, 20% methanol, 25 mM Tris) from the polyacrylamide gel to a nitrocellulose membrane (manufactured by Bio-Rad) using 200 mA conditions for 90 minutes at 4 °C using gel membrane transcription equipment (manufactured by Marisol, NP7513).

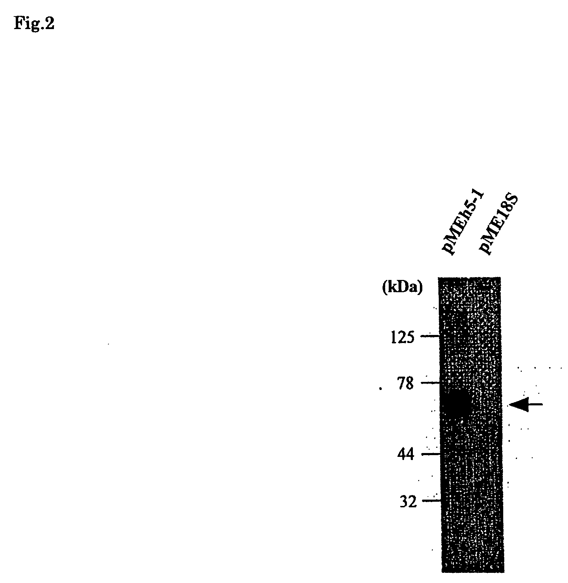

As for the nitrocellulose membrane after transcription, Western-blotting analysis using the antibody (hereinafter referred to as "55-1-N1 antibody" or "55-1-C1 antibody") obtained in Example 2 was performed. That is, the nitrocellulose membrane is first washed with PBS containing 0.05 % of Tween 20 (hereinafter referred to as "0.05% Tween 20-PBS") (at room temperature for 15 minutes once and then for 5 minutes twice). Then, it was put into a plastic bag (brand name hybribag, manufactured by Cosmobio), and 20 ml of 0.05% Tween 20-PBS(s) which contained 5 % skimmed milk (Snow Brand Milk Products CO., LTD.) were added, and incubated at room temperature for 1 hour. After one hour, the membrane was taken out, and washed for 15 minutes once, subsequently for 5 minutes twice with 0.05% Tween 20-PBS. After washing, the membrane was transferred to a new plastic bag, and incubated after adding 20 ml of 0.05% Tween 20-PBS containing 55-1-N1 antibody or 55-1-C1 antibody (100 times dilution) and 1% bovine serum albumin (hereinafter referred to as "BSA", manufactured by Sigma). One hour later, the membrane was taken out, washed for 15 minutes once, subsequently for 5 minutes twice with 0.05% Tween 20-PBS. Then, the membrane was transferred to a new plastic bag, to which was added 20 ml of a solution obtained by diluting 2000-fold western horseradish-peroxidase labeled anti-rabbit IgG antibody with 0.05 % Tween 20-PBS containing 1 % BSA, and incubated at a room temperature for 1 hour. One hour later, the membrane was taken out, and washed with a 0.05 % Tween 20-PBS solution for 15 minutes once, 5 minutes four times. After washing, the membrane was placed on a wrap film, and the band to which 55-1-N1 antibody or 55-1-C1 antibody binds was detected using ECL Western-blotting detection solution (manufactured by Amersham Pharmacia ) (leaving it at room temperature for 1 hour, after placing the membrane on a wrap film and dipping it in an ECL Western-blotting detection solution for 1 minute. Thereby, the background was attenuated, and the X-ray film was exposed to it (for 3 seconds)). Consequently, the specific band in the COS-1 culture supernatant to which the pMEh55-1 plasmid DNA was the was detected with both antibodies (Fig. 2).

The same experiment can be conducted also as for the COS-1 cell-culture supernatant wherein the mouse cDNA obtained in Reference Example 1 is expressed. That is, the insert DNA fragment of about 1.6 kb(s) obtained by digesting phagemid #55-1 DNA obtained in Reference example 1 with restriction enzymes EcoRI and XbaI is incorporated in pME18S to construct a clone (pME55-1), which is then introduced into COS-1 cells by the same method as above, and the culture supernatant is collected. When the sample prepared from this culture supernatant was analyzed by Western blotting using 55-1-N1 antibody and 55-1-C1 antibody, a specific band was detected in the COS-1 culture supernatant obtained by introducing pME55-1 plasmid DNA in any case of using either antibody for detection.

In the above-mentioned experimental system, the effect as a therapeutic or preventive agent for hyperlipidemia of a test substance can be investigated by preparing a sample from the culture supernatant of a KK mouse primary-culture hepatocyte cultured in the presence or absence of the test substance, and performing the same operation. A test substance which reduces the amount of detected antigen in this experiment may serve as a therapeutic or preventive agent for hyperlipidemia. When performing a multi-test substance treatment, the electrophoresis can be omitted and a dot blot and a slot blot can be performed.

### Example 4. Preparation of recombinant adenovirus and expression thereof in culture cells

In order to carry out forcible expression of the cDNA originating from the mouse obtained in Reference example 1, a recombinant adenovirus was produced using a commercial kit (an adenovirus expression vector kit, TAKARA SHUZO CO., LTD.). Namely, the phagemid clone #55-1 obtained in Reference example 1 was digested with restriction enzymes EcoRI and NotI, and the ends of the obtained DNA fragment of about 1.7 kb(s) were blunted, and it was used for the following steps as an insert DNA fragment.

Moreover, a cosmid pAxCA/mAP5 wherein the insert DNA fragment was incorporated at the restriction enzyme SwaI recognition site of the cosmid vector pAxCAwt (appended to an adenovirus expression vector kit) was designed so that expression may be conducted using a cytomegalovirus enhancer and a fowl β-actin promoter. pAxCA/mAP5 DNA and an end protein binding virus DNA (appended to DNA-TPC, adenovirus expression vector kit) were co-transfected to the 293 cells (ATCC CRL1573) using a calcium phosphate transfection system (manufactured by Lifetech), to isolate a recombinant adenovirus Ad/mAP-5, and proliferated in 293 cells. Moreover, adenovirus Ad/LacZ carrying a recombinant adenovirus vector into which the LacZ gene cut out from a control cosmid pAxCAiLacZ was incorporated was produced as well, and proliferated in the 293 cells. The proliferated virus was recovered from the 293 cells by conducting ultrasonication four times on the 293 cells infected with the virus for 30 seconds (B-1200, manufactured by Branson) and then repeating purification by cesium-chloride density-gradient centrifugation twice. The obtained virus liquid was dialyzed at 4 °C with PBS to which 10 % glycerol was added, and frozen and saved at - 70 °C or lower until it was used.

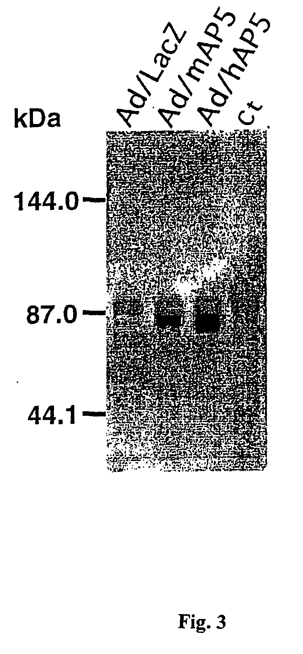

HeLa cells (ATCC CCL2) were infected with the thus obtained recombinant adenovirus at about 5 m.o.i. (multiplicity of infection), cultured in a serum free medium (Dulbecco's modified Eagle culture medium (DMEM)) for three to four days, and then the culture supernatant was collected. One ml of the supernatant was put into a 1.5 ml volume Eppendorf tube, to which 100 µl of trichloroacetic acid were added, and then left at a room temperature for 3 minutes, and then centrifuged at 15000 rpm with a desk centrifuge apparatus for 5 minutes. The supernatant was removed, 0.5 ml of acetone cooled with ice was added, and stirred well, and it was then centrifuged again at 15000 rpm with a desk centrifuge apparatus for 2 minutes. After removing the supernatant, 0.5 ml of acetone cooled with ice were added again, agitated and centrifuged for 2 minutes at 15000 rpm(s) with the desk centrifuge apparatus. Then, the supernatant was removed, and the precipitate was dried using vacuum evaporation. The precipitate was dissolved in 10 µl of distilled water, mixed with a SDS-PAGE sample buffer solution (manufactured by Bio-Rad) containing 2-mercaptoethanol, heated for 5 minutes at 99 °C, and thereby the sample for electrophoresis was prepared. The sample was subjected to electrophoresis with polyacrylamide density-gradient gel at a gel concentration of 4-20% (buffer solution for electrophoresis: 25 mM Tris, 192 mM glycine, and 0.1% SDS), and then transferred to a nitrocellulose membrane in a transferring buffer solution at 200 mA and 4°C for 1 hour. The transferred membrane was blocked at 4 °C overnight with PBS to which 0.5 % of skimmed milk was added, and then washed three times with a washing solution (0.05% Tween 20-PBS). Subsequently, after putting the membrane into the reaction mixture obtained by mixing the antiserum of 55-1-N1 antibody before purification and the antiserum of 55-1-C1 antibody before purification, this was then diluted 10000 times with 0.05% Tween 20-PBS which contained 5 % fetal bovine serum, and then incubated at a room temperature for 1 hour, followed by 3 times of washing with the washing solution. Furthermore, the membrane was incubated in a reaction mixture obtained by diluting 10000 times western horseradish-peroxidase labelled goat anti-rabbit IgG (H+L) (manufactured by Bio-Rad) with 0.05% Tween 20-PBS which contained 5 % fetal bovine serum at room temperature for 1 hour, and then washed 5 times with the washing solution. The membrane was placed on a wrap film, and immersed in an ECL Western-blotting detection solution for 1 minute, then left at room temperature for 1 hour to attenuate the background, and then an X-ray film was exposed to it (for 3 seconds). Consequently, the specific band in the lane of the sample originating from HeLa-cells culture supernatant infected with a recombinant adenovirus Ad/mAP-5 was detected (Fig. 3).

On the other hand, a recombinant adenovirus (Ad/hAP5) which has an adenovirus vector in which cDNA carried by the phagemid clone #h5-1 was incorporated was prepared, and subjected to a similar experiment. In Western blotting analysis of the culture supernatant expressed in HeLa cells, a specific band was detected as well. (Fig. 3).

### Example 5. Expression in vivo using a recombinant adenovirus

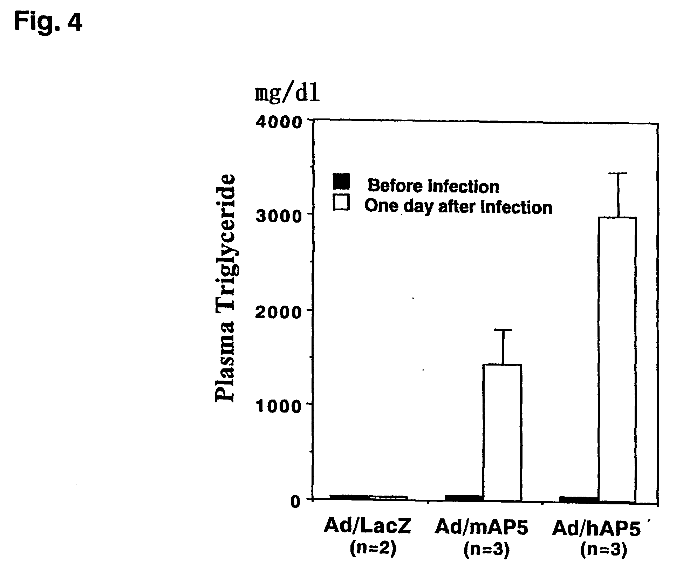

The recombinant adenovirus Ad/mAP-5 or Ad/LacZ purified as mentioned above was diluted with PBS which contained 10 % glycerol to give 2 x 10¹⁰ pfu (plaque forming unit)/ml, and 100 µl (2x10⁹ pfu) respectively, and each of them was inoculated into three (Ad/mAP -5) or two (Ad/LacZ) 13 -14-week old male KK/San mice by tail intravenous injection. One day after inoculation, blood was collected from the orbit of each mouse with a hematocrit pipe, centrifuged at 5200 rpm(s) with a desk centrifuge apparatus for 15 minutes to separate plasma. Then, neutral-fat concentration was measured using a kit for neutral-fat measurement (triglyceride E-test Wako, Wako Pharmaceuticals). In the Ad/LacZ inoculated mouse group, there was no significant difference in neutral-fat concentration in blood, whereas a significant difference in neutral-fat concentration in blood (Fig. 4) was detected between an Ad/mAP-5 inoculated mouse group and the other two groups. (Fig.4).

As for the recombinant adenovirus (Ad/hAP5) which has an adenovirus vector in which cDNA carried by the phagemid clone #h5-1 is incorporated, the same experiment was conducted. Namely, it was expressed in a male KK/San mouse. As a result, significant elevation of neutral-fat concentration in blood was seen, and it became clear that the human type molecule functions also in a mouse (Fig. 4).

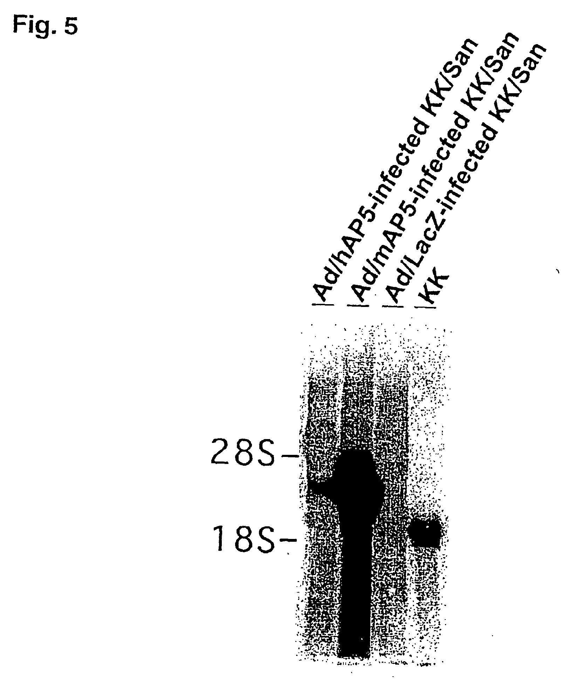

Moreover, when total RNA was collected from the liver of the mouse group infected with the adenovirus in which significant difference was detected in neutral-fat concentration in blood, and subjected to Northern blotting analysis according to the method described in Example 1, it was confirmed that the introduced gene was actually expressed highly (Fig. 5). Although the band detected in the KK/San mouse group infected with the adenovirus is larger than the band detected in the KK mouse which is not genetically manipulated, it is considered that an effective transcription initiation site or the (Poly A) addition signal in a recombinant adenovirus vector are different from the original gene. Anyway, the difference in the size of this mRNA does not influence the amino acid sequence translated, since the translation termination codon exists just the 5'-end side of the first translation initiation codon in cDNA incorporated in the recombinant adenovirus vector in the same reading frame.

### Example 6: Purification of recombinant protein and determination of N-terminus amino acid sequence

According to the following method, the transformation of the animal cell was carried out using the expression vector pMEh55-1 which was created in Example 3 and in which the human cDNA having the nucleotide sequence shown in SEQ ID No. 3 of the Sequence Listing was incorporated, the recombinant protein secreted in the culture supernatant of the transformed cell was purified, and the N-terminus amino acid sequence thereof was determined.
(1) Acquisition of a transformant, and preparation of a culture supernatant
   Dyhydrofolic-acid reductase deficit CHO cells (ATCC CRL-9096) were proliferated in αMEM (manufactured by Gibco BRL) containing 10 % FCS (manufactured by Gibco BRL), 10 units/ml of penicillin and 10 µg/ml of streptomycin (product manufactured by the Gibco BRL), and then transfected with pMEh55-1 plasmid at a rate of 1 µg/10⁶ cells using a transfection reagent (FuGENE6: manufactured by Roche Diagnostics). Specifically, the cells were cultured at 1.5 x 10⁷ cells/laboratory dish on 200 laboratory dishes for cell cultures (150 mmφ, manufactured by Coning). For transfection, 15 µg of pMEh55-1 plasmid per laboratory dish were used. After transfection, the cells were cultured for 24 hours in the culture medium which contains the above FCS, and the medium was replaced by serum-free αMEM (30 ml/laboratory dish). Three more days after exchanging the culture medium, 6 l of serum-free supernatant were collected.
(2) Purification of recombinant protein
   1) To 1.6 l of SEPHADEX G25 (manufactured by Amersham Pharmacia biotech) with which a column (stream line C-100 : manufactured by Amersham Pharmacia biotech) was filled up was poured 600 ml of the culture supernatant obtained above 1) (flow rate: 20 ml/min). Subsequently, an elution buffer solution (20 mM Tris-hydrochloric acid (pH 7.5), 0.01% sodium azide (manufactured by Sigma), 0.05% protease-inhibitor mixture (manufactured by Sigma), 0.05% Tween 20 (manufactured by Sigma))(hereinafter referred to as "Liquid A") was passed through the column at a flow rate of 50 ml/min, and 1500 ml of the first eluates were collected. The operation was carried out 10 times, and thereby desalting and removal of low weight molecules from the 6 l of the culture supernatant obtained in the above 1) were performed.
   2) Next, the eluate obtained by the above 1) was fractionalized using FPLC equipment (Biopilot system, manufactured by Amersham Pharmacia biotech) according to the ion exchange chromatography under the following conditions. Column: XK50/100 column (manufactured by Amersham Pharmacia biotech) was filled up with 100 ml of Q Sepharose fastflow (manufactured by Amersham Pharmacia biotech).
      Elution buffer-solution composition:
      [Liquid A] above reference
      [Liquid B] 20 mM Tris-hydrochloric acid, 1 M sodium chloride (pH 7.5), 0.01 % sodium azide, 0.05 % protease-inhibitor mixture, 0.05 % Tween 20
      Flow Rate: 10 ml/min
      Fractionalization: 10 ml/tube
      Temperature: 4 °C
      Elution conditions: linear-gradient from Liquid A 100% to Liquid B 100% (for 60 minutes), the fraction eluted at a sodium chloride concentration of 0.3 - 0.4 M was collected.
   3) The fraction collected by the ion exchange chromatography in the above 2) was subjected to group specific affinity chromatography under the following conditions using FPLC equipment.
      Column: XK16/40 column (manufactured by Amersham Pharmacia biotech) was filled up with 10 ml of Affigel blue (manufactured by Bio-Rad).
      Elution buffer solution composition :
         [Liquid C] 20 mM Tris- hydrochloric acid, 0.5M sodium chloride (pH 7.5), 0.01% sodium azide, 0.05% protease-inhibitor mixture, 0.05% Tween 20
         [Liquid D] 20 mM Tris- hydrochloric acid, 1M sodium chloride (pH 7.5), 0.01% sodium azide, 0.05% protease-inhibitor mixture, 0.05% Tween 20
      Flow Rate: 1.5 ml/min
      Temperature: 4 °C

      After passing the fraction collected in the above 2) through the column, 60 ml of Liquid C were passed through to wash, 100 ml of Liquid D were poured, and eluates with D liquid were collected.
   4) An equal amount (100 ml) of Liquid A was added to the eluate obtained by the above 3). It was subjected to a group specific affinity chromatography under the following conditions indicated below using FPLC equipment.
      Column: XK16/40 column was filled up with 10 ml of lentil lectin Sepharoses 4B (manufactured by Amersham Pharmacia biotech).
      Elution buffer-solution composition:
         [C liquid] see the above description
         [F liquid] 20 mM Tris- hydrochloric acid, 0.5 M sodium chloride, 0.3 M methyl mannopyranoside (pH 7.5), 0.01 % sodium azide, 0.05 % protease-inhibitor mixture, 0.05% Tween 20
      Flow Rate: 1 ml/min
      Temperature: 4 °C

   After passing the sample through the column, 50 ml of Liquid C were passed to wash, 50 ml of Liquid F were passed and eluate with Liquid F was collected.
   The eluate was transferred to a dialysis tube (exclusion-limit molecular weight 10 KDa: manufactured by Gibco BRL), and it was dialyzed at 4 °C overnight to 2 l of Dulbecco's modified PBS(-) (manufactured by NISSUI PHARMACEUTICAL CO., LTD.) containing 0.01 % sodium azide and 0.1% protease-inhibitor mixture. Then, the solution in the dialysis tube was collected. To 4 ml of it was added 1/10 volume (0.4 ml) of TCA containing 4 mg/ml of sodium deoxycholate. The obtained precipitate was collected, and the precipitate obtained by adding acetone was collected, which was then dissolved in 50 µl of sterilized ultrapure water.
(3) Determination of the N terminus amino acid sequence
   PNGaseF (produced by New England Biolab) was added to the sample purified in the above (2), and the N binding type sugar chain was cut. Specifically, 6 µl of 10 x G7 buffer (appended to the above PNGaseF reagent) were added to 50 µl of the sample, then agitated, and heated in a boiling bath for 10 minutes. After cooling it to room temperature, 6 µl of 10% Nonidet P-40 of and 6 µl of 10 x G7 buffer (appended to the above PNGaseF reagent) were added in order, and it was agitated. 3 µl of the PNGaseF were added and stirred, and then the mixture was kept in a water bath at 37 °C for 2 hours or more.

As for the reaction mixture after this N binding type sugar-chain cutting reaction, SDS-PAGE was carried out under reduction conditions using 4 - 20 % concentration gradient acrylamide gel (Multi gel 4/20, manufactured by Daiichikagakuyakuhin) and mini slab electrophoresis equipment (manufactured by Nihoneido). After electrophoresis, the protein separated in the gel was transferred to a poly vinylidene difluoride (PVDF) film (manufactured by Bio-Rad ) having a pore size of 0.2 µm (2 mA/cm², 4 °C, for 2 hours) using gel membrane transcription equipment (manufactured by Marisol). After transcription, the film was soaked in 100% methanol (Wako Pure Chem ) for 10 seconds, washed for 2 minutes with ultrapure water, dyed with a Coomasie staining solution (manufactured by Bio-Rad) for 5 minutes, and then the membrane was immersed in methanol for two hours for decolorizing. As a result, a band which is equivalent to about 50 KDa was seen. A part of the band of the film was cut out and the N terminus sequence was analyzed in a protein sequencer (PPSQ-10, manufactured by Shimadzu Seisakusho).

Consequently, the N terminus amino acid sequence of the above-mentioned band of about 50 kDa was as follows:
Ser-Arg-Ile-Asp-Gln-Asp-Asn-Ser-Ser-Phe-Asp (amino acid numbers 17 to 27 of SEQ ID No. 4 of the Sequence Listing)

Therefore, protein encoded by the nucleotide sequence shown in the nucleotide numbers 78-1457 of SEQ ID No. 3 of the Sequence Listing was secreted, in the mammalian cell, after 16 residues from the N terminus (amino acid numbers 1-16 of SEQ ID No. 4 of the Sequence Listing) were cut off, as a mature protein of which N terminus is serine residue which continues immediately after it.

### [Industrial Applicability]

As described above, according to the present invention, it was confirmed that the genes consisting of the nucleotide sequence shown in the nucleotide numbers 47-1411 of SEQ ID No. 1 of the Sequence Listing and the nucleotide sequence shown in the nucleotide numbers 78-1457 of SEQ ID No. 3 of the Sequence Listing are hereditary diatheses which raise neutral-fat concentration in blood, and therefore substances which control the level of expression of these genes may serve as a therapeutic or preventive agent for hyperlipidemia. That is, the method of the present invention, the polynucleotide used as a probe or a primer in the mode which detects a nucleic acid among the methods, and the antibody used in the mode which similarly detects the polypeptide are useful for treatment of hyperlipidemia, or search for a therapeutic or preventive agent for hyperlipidemia.

## Claims

1. A method for testing an effect of a substance as a therapeutic or preventive agent for hyperlipidemia comprising the following steps:
1) culturing cells in the presence or absence of a test substance;
2) detecting the amount of expression of mRNA which has a nucleotide sequence of any one of the following sequences a) to e) (however, t in the sequence is read as u) in the cultured cells obtained in the above 1):
a) the nucleotide sequence shown in the nucleotide numbers 47-1411 of SEQ ID No. 1 of the Sequence Listing;
b) the nucleotide sequence shown in the nucleotide numbers 78-1457 of SEQ ID No. 3 of the Sequence Listing;
c) the nucleotide sequence of DNA incorporated into a phagemid carried by transformed Escherichia coli E.coli pBK/m55-1 SANK72199 (FERM BP-6940);
d) the nucleotide sequence of DNA incorporated into a phagemid carried by transformed Escherichia coli E.coli pTrip/h55-1 SANK72299 (FERM BP-6941);
e) the nucleotide sequence which hybridizes with the polynucleotide consisting of an antisense sequence of the nucleotide sequence described in any one of the above a) to d) under stringent conditions, and encodes a polypeptide having the activity of raising neutral fat concentration in blood; and
3) comparing the amount of expression of mRNA between the cells cultured in the absence of the substance and the cells cultured in the presence of the substance, as a result of detection in the above mentioned step 2).

2. The method according to Claim 1 wherein the cultured cell originates from liver.

3. The method according to Claim 1 or 2 wherein the cultured cell originates from a primate or a rodent animal.

4. The method according to Claim 3 wherein the cultured cell originates from a human or a mouse.

5. The method according to any one of Claims 1 to 4 wherein the method for detecting the amount of mRNA expressed is Northern blotting, dot blotting, or slot blotting.

6. The method according to any one of Claims 1 to 4 wherein the method for detecting the amount of mRNA expressed is RT-PCR.

7. The method according to any one of Claims 1 to 4 wherein the method for detecting the amount of mRNA expressed is ribonuclease protection assay.

8. The method according to any one of Claims 1 to 4 wherein the method for detecting the amount of mRNA expressed is run-on assay.

9. A polynucleotide having a nucleotide sequence hybridizing to mRNA containing any one nucleotide sequence selected from the following a) to d) (however, t in the sequence is read as u) under stringent conditions [excluding those containing the nucleotide sequence disclosed in the following a) to d)]:
a) the nucleotide sequence shown in the nucleotide numbers 47-1411 of SEQ ID No. 1 of the Sequence Listing;
b) the nucleotide sequence shown in the nucleotide numbers 78-1457 of SEQ ID No. 3 of the Sequence Listing;
c) the nucleotide sequence of DNA incorporated into a phagemid carried by transformed Escherichia coli E.coli pBK/m55-1 SANK72199 (FERM BP-6940);
d) the nucleotide sequence of DNA incorporated into a phagemid carried by transformed Escherichia coli E.coli pTrip/h55-1 SANK72299 (PERM BP-6941).

10. DNA consisting of at least 15 nucleotides of the nucleotide sequence shown in the nucleotide numbers 47-1411 of SEQ ID No. 1 of the Sequence Listing wherein one or more of nucleotides are deleted at one end or both ends thereof.

11. DNA consisting of at least 15 nucleotides of the nucleotide sequence shown in the nucleotide numbers 78-1457 of SEQ ID No. 3 of the Sequence Listing wherein one or more of nucleotides are deleted at one end or both ends.

12. A method for testing the effect of a substance as a therapeutic or preventive agent for hyperlipidemia comprising the following steps:
1) culturing cells in the presence or absence of the test substance;
2) detecting the amount of production of polypeptide having an amino acid sequence encoded by the nucleotide sequence of any one of the following sequences a) to e) or a part thereof in the supernatant of the cultured cells obtained in the above 1) using an antibody specifically recognizing the polypeptide;
a) the nucleotide sequence shown in the nucleotide numbers 47-1411 of SEQ ID No. 1 of the Sequence Listing;
b) the nucleotide sequence shown in the nucleotide numbers 78-1457 of SEQ ID No. 3 of the Sequence Listing;
c) the nucleotide sequence of DNA incorporated into a phagemid carried by transformed Escherichia coli E.coli pBK/m55-1 SANK72199 (FERM BP-6940);
d) the nucleotide sequence of DNA incorporated into a phagemid carried by transformed Escherichia coli E.coli pTrip/h55-1 SANK72299 (FERM BP-6941);
e) a nucleotide sequence which hybridizes with a polynucleotide consisting of an antisense sequence of the nucleotide sequence described in any one of the above a) to d) under stringent conditions, and encodes a polypeptide having an activity of raising neutral fat concentration in blood; and
3) comparing the amount of production of the polypeptide between the cells cultured in the absence of the substance and the cells cultured in the presence of the substance, as a result of that detected in the above mentioned step 2).

13. The method according to Claim 12 wherein the antibody which specifically recognizes a polypeptide consisting of the amino acid sequence encoded by any one of the sequences a) to e) or a part thereof is an antibody which specifically recognizes a polypeptide which consists of the amino acid sequence shown in the amino acid number 17-455 of SEQ ID No. 2 of the Sequence Listing or a part thereof, a polypeptide which consists of the amino acid sequence shown in 19-455 of the same sequence as the above or a part thereof, or a polypeptide which consists of the amino acid sequence shown in the amino acid number 17-460 of SEQ ID No. 4 of the Sequence Listing or a part thereof.

14. The method according to Claim 12 or 13 wherein the antibody which specifically recognizes a polypeptide consisting of the amino acid sequence encoded by any one of the sequences a) to e) or a part thereof is an antibody which specifically recognizes the amino acid sequence shown in the amino acid sequence shown in the amino acid numbers 1-13 of SEQ ID No. 9 or the amino acid numbers 1-14 of SEQ ID No. 10 of the Sequence Listing.

15. The method according to any one of Claims 12 to 14 wherein the method for detecting using the antibody which specifically recognizes a polypeptide consisting of the amino acid sequence encoded by any one of the sequences a) to e) or a part thereof is Western blotting, a dot blotting or a slot blotting.

16. The method according to any one of Claims 12 to 14 wherein the method for detecting using the antibody which specifically recognizes a polypeptide consisting of the amino acid sequence encoded by any one of the sequences a) to e) or a part thereof is a solid-phase enzyme immunoassay (the ELISA method), or a radioisotope immunoassay (the RIA method).

17. An antibody which specifically recognizes a polypeptide consisting of the amino acid sequence encoded by any one of the following sequences a) to e) or a part thereof:
a) the nucleotide sequence shown in the nucleotide numbers 47-1411 of SEQ ID No. 1 of the Sequence Listing;
b) the nucleotide sequence shown in the nucleotide numbers 78-1457 of SEQ ID No. 3 of the Sequence Listing;
c) the nucleotide sequence of DNA incorporated into a phagemid carried by transformed Escherichia coli E.coli pBK/m55-1 SANK72199 (FERM BP-6940);
d) the nucleotide sequence of DNA incorporated into a phagemid carried by transformed Escherichia coli E.coli pTrip/h55-1 SANK72299 (FERM BP-6941);
e) the nucleotide sequence which hybridizes with a polynucleotide consisting of an antisense sequence of the nucleotide sequence described in any one of the above a) to d) under stringent conditions, and encodes a polypeptide having an activity of raising neutral fat concentration in blood.

18. The antibody according to Claim 17 wherein the antibody specifically recognizes the amino acid sequence shown in the amino acid sequence shown in the amino acid numbers 1-13 of SEQ ID No. 9 or the amino acid numbers 1-14 of SEQ ID No. 10 of the Sequence Listing.

19. A kit for testing a therapeutic or preventive agent for hyperlipidemia which comprises the antibody of claim 17 or 18.

20. A method for testing the effect of a substance as a therapeutic or preventive agent for hyperlipidemia comprising the following steps:
1) administrating the substance to be tested to an animal other than a human obtained by genetic manipulation in which a foreign gene containing the nucleotide sequence of any one of those shown in the following a) to e) is introduced so that the gene can be highly expressed;
a) the nucleotide sequence shown in the nucleotide numbers 47-1411 of SEQ ID No. 1 of the Sequence Listing;
b) the nucleotide sequence shown in the nucleotide numbers 78-1457 of SEQ ID No. 3 of the Sequence Listing;
c) the nucleotide sequence of DNA incorporated into a phagemid carried by transformed Escherichia coli E.coli pBK/m55-1 SANK72199 (FERM BP-6940);
d) the nucleotide sequence of DNA incorporated into a phagemid carried by transformed Escherichia coli E.coli pTrip/h55-1 SANK72299 (FERM BP-6941);
e) the nucleotide sequence which hybridizes with a polynucleotide consisting of an antisense sequence of the nucleotide sequence described in any one of the above a) to d) under stringent conditions, and encodes a polypeptide having an activity of raising neutral fat concentration in blood; and
2) measuring the concentration of neutral-fat in the blood of the animal shown in 1).

21. The method according to Claim 20 wherein the animal other than human in 1) is a mouse.

22. The method according to Claim 20 wherein the method for introducing the foreign gene to the non-human animal is the method of infecting the animal with an adenovirus which contains an adenovirus vector in which the gene was introduced.

23. Use for the manufacture of a probe used for the treatment of hyperlipidemia of polynucleotide having a nucleotide sequence which hybridizes under stringent conditions with mRNA comprising the nucleotide sequence of any one of the following sequences a) to e) (however, t in the sequence is read as u):
a) the nucleotide sequence shown in the nucleotide numbers 47-1411 of SEQ ID No. 1 of the Sequence Listing;
b) the nucleotide sequence shown in the nucleotide numbers 78-1457 of SEQ ID No. 3 of the Sequence Listing;
c) the nucleotide sequence of DNA incorporated into a phagemid carried by transformed Escherichia coli E.coli pBK/m55-1 SANK72199 (FERM BP-6940);
d) the nucleotide sequence of DNA incorporated into a phagemid carried by transformed Escherichia coli E.coli pTrip/h55-1 SANK72299 (FERM BP-6941).

24. Use for producing an antibody used for testing a therapeutic or preventive agent for hyperlipidemia of a polypeptide consisting of the amino acid sequence shown in the amino acid number 17-455 of SEQ ID No. 2 of the Sequence Listing or a part thereof, a polypeptide which consists of the amino acid sequence shown in 19-455 of the same sequence as the above or a part thereof, or a polypeptide which consists of the amino acid sequence shown in the amino acid number 17-460 of SEQ ID No. 4 of the Sequence Listing or a part thereof.

25. Use for producing a test kit for a therapeutic or preventive agent for hyperlipidemia of an antibody that specifically recognizes a polypeptide consisting of the amino acid sequence shown in the amino acid number 17-455 of SEQ ID No. 2 of the Sequence Listing or a part thereof, a polypeptide which consists of the amino acid sequence shown in 19-455 of the same sequence as the above or a part thereof, or a polypeptide which consists of the amino acid sequence shown in the amino acid number 17-460 of SEQ ID No. 4 of the Sequence Listing or a part thereof.

26. DNA or RNA consisting of an antisense sequence of the nucleotide sequence which consists of a continuous 15 or 30 nucleotides in the nucleotide sequence shown in the nucleotide numbers 78-1457 of SEQ ID No. 3 of the Sequence Listing.

27. A therapeutic agent for hyperlipidemia containing the DNA or RNA of Claim 26.


SEQUENCE LISTING

<110> Sankyo Company, Limited

<120> Method of Testing Anti-hyperlipidemia Substances

<130> EPP83633

<140> WO0142499

<141> 2001-06-14

<150> JP 1999 0349976

<151> 1999-12-09

<160> 11

<170> PatentIn version 3.1

<210> 1

<211> 1604

<212> DNA

<213> Mus musculus

<220>

<221> CDS

<222> (47)..(1411)

<223>

<400> 1
ggcacgaggt tccaaattgc ttaaaattga ataattgaga caaaaa atg cac aca 55
Met His Thr
1

att aaa tta ttc ctt ttt gtt gtt cct tta gta att gca tcc aga gtg 103
Ile Lys Leu Phe Leu Phe Val Val Pro Leu Val Ile Ala Ser Arg Val
    5                   10                  15

gat cca gac ctt tca tca ttt gat tct gca cct tca gag cca aaa tca 151
Asp Pro Asp Leu Ser Ser Phe Asp Ser Ala Pro Ser Glu Pro Lys Ser
20                  25                  30                  35

aga ttt gct atg ttg gat gat gtc aaa att tta gcg aat ggc ctc ctg 199
Arg Phe Ala Met Leu Asp Asp Val Lys Ile Leu Ala Asn Gly Leu Leu
                40                  45                  50

cag ctg ggt cat gga ctt aaa gat ttt gtc cat aag act aag gga caa 247
Gln Leu Gly His Gly Leu Lys Asp Phe Val His Lys Thr Lys Gly Gln
            55                  60                  65

att aac gac ata ttt cag aag ctc aac ata ttt gat cag tct ttt tat 295
Ile Asn Asp Ile Phe Gln Lys Leu Asn Ile Phe Asp Gln Ser Phe Tyr
        70                  75                  80

gac cta tca ctt cga acc aat gaa atc aaa gaa gag gaa aag gag cta 343
Asp Leu Ser Leu Arg Thr Asn Glu Ile Lys Glu Glu Glu Lys Glu Leu
    85                  90                  95

aga aga act aca tct aca cta caa gtt aaa aac gag gag gtg aag aac 391
Arg Arg Thr Thr Ser Thr Leu Gln Val Lys Asn Glu Glu Val Lys Asn
100                 105                 110                 115

atg tca gta gaa ctg aac tca aag ctt gag agt ctg ctg gaa gag aag 439
Met Ser Val Glu Leu Asn Ser Lys Leu Glu Ser Leu Leu Glu Glu Lys
                120                 125                 130

aca gcc ctt caa cac aag gtc agg gct ttg gag gag cag cta acc aac 487
Thr Ala Leu Gln His Lys Val Arg Ala Leu Glu Glu Gln Leu Thr Asn
            135                 140                 145

tta att cta agc cca gct ggg gct cag gag cac cca gaa gta aca tca 535
Leu Ile Leu Ser Pro Ala Gly Ala Gln Glu His Pro Glu Val Thr Ser
        150                 155                 160

ctc aaa agt ttt gta gaa cag caa gac aac agc ata aga gaa ctc ctc 583
Leu Lys Ser Phe Val Glu Gln Gln Asp Asn Ser Ile Arg Glu Leu Leu
    165                 170                 175

cag agt gtg gaa gaa cag tat aaa caa tta agt caa cag cac atg cag 631
Gln Ser Val Glu Glu Gln Tyr Lys Gln Leu Ser Gln Gln His Met Gln
180                 185                 190                 195

ata aaa gaa ata gaa aag cag ctc aga aag act ggt att caa gaa ccc 679
Ile Lys Glu Ile Glu Lys Gln Leu Arg Lys Thr Gly Ile Gln Glu Pro
                200                 205                 210

tca gaa aat tct ctt tct tct aaa tca aga gca cca aga act act ccc 727
Ser Glu Asn Ser Leu Ser Ser Lys Ser Arg Ala Pro Arg Thr Thr Pro
            215                 220                 225

cct ctt caa ctg aac gaa aca gaa aat aca gaa caa gat gac ctt cct 775
Pro Leu Gln Leu Asn Glu Thr Glu Asn Thr Glu Gln Asp Asp Leu Pro
        230                 235                 240

gcc gac tgc tct gcc gtt tat aac aga ggc gaa cat aca agt ggc gtg 823
Ala Asp Cys Ser Ala Val Tyr Asn Arg Gly Glu His Thr Ser Gly Val
    245                 250                 255

tac act att aaa cca aga aac tcc caa ggg ttt aat gtc tac tgt gat 871
Tyr Thr Ile Lys Pro Arg Asn Ser Gln Gly Phe Asn Val Tyr Cys Asp

260                 265                 270                 275

acc caa tca ggc agt cca tgg aca tta att caa cac cgg aaa gat ggc      919
Thr Gln Ser Gly Ser Pro Trp Thr Leu Ile Gln His Arg Lys Asp Gly
                280                 285                 290

tca cag gac ttc aac gaa aca tgg gaa aac tac gaa aag ggc ttt ggg      967
Ser Gln Asp Phe Asn Glu Thr Trp Glu Asn Tyr Glu Lys Gly Phe Gly
            295                 300                 305

agg ctc gat gga gaa ttt tgg ttg ggc cta gag aag atc tat gct ata      1015
Arg Leu Asp Gly Glu Phe Trp Leu Gly Leu Glu Lys Ile Tyr Ala Ile
        310                 315                 320

gtc caa cag tct aac tac att tta cga ctc gag cta caa gac tgg aaa      1063
Val Gln Gln Ser Asn Tyr Ile Leu Arg Leu Glu Leu Gln Asp Trp Lys
    325                 330                 335

gac agc aag cac tac gtt gaa tac tcc ttt cac ctg ggc agt cac gaa      1111
Asp Ser Lys His Tyr Val Glu Tyr Ser Phe His Leu Gly Ser His Glu
340                 345                 350                 355

acc aac tac acg cta cat gtg gct gag att gct ggc aat atc cct ggg      1159
Thr Asn Tyr Thr Leu His Val Ala Glu Ile Ala Gly Asn Ile Pro Gly
                360                 365                 370

gcc ctc cca gag cac aca gac ctg atg ttt tct aca tgg aat cac aga      1207
Ala Leu Pro Glu His Thr Asp Leu Met Phe Ser Thr Trp Asn His Arg
            375                 380                 385

gca aag gga cag ctc tac tgt cca gaa agt tac tca ggt ggc tgg tgg      1255
Ala Lys Gly Gln Leu Tyr Cys Pro Glu Ser Tyr Ser Gly Gly Trp Trp
        390                 395                 400

tgg aat gac ata tgt gga gaa aac aac cta aat gga aaa tac aac aaa      1303
Trp Asn Asp Ile Cys Gly Glu Asn Asn Leu Asn Gly Lys Tyr Asn Lys
    405                 410                 415

ccc aga acc aaa tcc aga cca gag aga aga aga ggg atc tac tgg aga      1351
Pro Arg Thr Lys Ser Arg Pro Glu Arg Arg Arg Gly Ile Tyr Trp Arg
420                 425                 430                 435

cct cag agc aga aag ctc tat gct atc aaa tca tcc aaa atg atg ctc      1399
Pro Gln Ser Arg Lys Leu Tyr Ala Ile Lys Ser Ser Lys Met Met Leu
                440                 445                 450

cag ccc acc acc taagaagctt caactgaact gagacaaaat aaaagatcaa      1451
Gln Pro Thr Thr
            455

taaattaaat attaaagtcc tcccgatcac tgtagtaatc tggtattaaa attttaatgg      1511

aaagcttgag aattgaattt caattaggtt taaactcatt gttaagatca gatatcaccg      1571

aatcaacgta aacaaaattt atctttttca atc      1604

<210>  2

<211>  455

<212>  PRT

<213>  Mus musculus

<400>  2

Met His Thr Ile Lys Leu Phe Leu Phe Val Val Pro Leu Val Ile Ala
1               5                   10                  15

Ser Arg Val Asp Pro Asp Leu Ser Ser Phe Asp Ser Ala Pro Ser Glu
            20                  25                  30

Pro Lys Ser Arg Phe Ala Met Leu Asp Asp Val Lys Ile Leu Ala Asn
        35                  40                  45

Gly Leu Leu Gln Leu Gly His Gly Leu Lys Asp Phe Val His Lys Thr
    50                  55                  60

Lys Gly Gln Ile Asn Asp Ile Phe Gln Lys Leu Asn Ile Phe Asp Gln
65                  70                  75                  80

Ser Phe Tyr Asp Leu Ser Leu Arg Thr Asn Glu Ile Lys Glu Glu Glu
                85                  90                  95

Lys Glu Leu Arg Arg Thr Thr Ser Thr Leu Gln Val Lys Asn Glu Glu
            100                 105                 110

Val Lys Asn Met Ser Val Glu Leu Asn Ser Lys Leu Glu Ser Leu Leu
        115                 120                 125

Glu Glu Lys Thr Ala Leu Gln His Lys Val Arg Ala Leu Glu Glu Gln
    130                 135                 140

Leu Thr Asn Leu Ile Leu Ser Pro Ala Gly Ala Gln Glu His Pro Glu
145                 150                 155                 160

Val Thr Ser Leu Lys Ser Phe Val Glu Gln Gln Asp Asn Ser Ile Arg
                165                 170                 175

Glu Leu Leu Gln Ser Val Glu Glu Gln Tyr Lys Gln Leu Ser Gln Gln
            180                 185                 190

His Met Gln Ile Lys Glu Ile Glu Lys Gln Leu Arg Lys Thr Gly Ile
        195                 200                 205

Gln Glu Pro Ser Glu Asn Ser Leu Ser Ser Lys Ser Arg Ala Pro Arg
    210                 215                 220

Thr Thr Pro Pro Leu Gln Leu Asn Glu Thr Glu Asn Thr Glu Gln Asp

225                 230                 235                 240

Asp Leu Pro Ala Asp Cys Ser Ala Val Tyr Asn Arg Gly Glu His Thr
                245                 250                 255

Ser Gly Val Tyr Thr Ile Lys Pro Arg Asn Ser Gln Gly Phe Asn Val
            260                 265                 270

Tyr Cys Asp Thr Gln Ser Gly Ser Pro Trp Thr Leu Ile Gln His Arg
        275                 280                 285

Lys Asp Gly Ser Gln Asp Phe Asn Glu Thr Trp Glu Asn Tyr Glu Lys
    290                 295                 300

Gly Phe Gly Arg Leu Asp Gly Glu Phe Trp Leu Gly Leu Glu Lys Ile
305                 310                 315                 320

Tyr Ala Ile Val Gln Gln Ser Asn Tyr Ile Leu Arg Leu Glu Leu Gln
                325                 330                 335

Asp Trp Lys Asp Ser Lys His Tyr Val Glu Tyr Ser Phe His Leu Gly
            340                 345                 350

Ser His Glu Thr Asn Tyr Thr Leu His Val Ala Glu Ile Ala Gly Asn
        355                 360                 365

Ile Pro Gly Ala Leu Pro Glu His Thr Asp Leu Met Phe Ser Thr Trp
    370                 375                 380

Asn His Arg Ala Lys Gly Gln Leu Tyr Cys Pro Glu Ser Tyr Ser Gly
385                 390                 395                 400

Gly Trp Trp Trp Asn Asp Ile Cys Gly Glu Asn Asn Leu Asn Gly Lys
                405                 410                 415

Tyr Asn Lys Pro Arg Thr Lys Ser Arg Pro Glu Arg Arg Arg Gly Ile
            420                 425                 430

Tyr Trp Arg Pro Gln Ser Arg Lys Leu Tyr Ala Ile Lys Ser Ser Lys
        435                 440                 445

Met Met Leu Gln Pro Thr Thr
    450                 455

<210>  3

<211>  1716

<212>  DNA

<213>  Mus musculus

<220>

<221>  CDS

<222>  (78)..(1457)

<223>

<400>  3
gcggccgcgt cgacgtctag gtctgcttcc agaagaaaac agttccacgt tgcttgaaat      60

tgaaaatcaa gataaaa atg ttc aca att aag ctc ctt ctt ttt att gtt      110
                   Met Phe Thr Ile Lys Leu Leu Leu Phe Ile Val
                   1               5                   10

cct cta gtt att tcc tcc aga att gat caa gac aat tca tca ttt gat      158
Pro Leu Val Ile Ser Ser Arg Ile Asp Gln Asp Asn Ser Ser Phe Asp
            15                  20                  25

tct cta tct cca gag cca aaa tca aga ttt gct atg tta gac gat gta      206
Ser Leu Ser Pro Glu Pro Lys Ser Arg Phe Ala Met Leu Asp Asp Val
        30                  35                  40

aaa att tta gcc aat ggc ctc ctt cag ttg gga cat ggt ctt aaa gac      254
Lys Ile Leu Ala Asn Gly Leu Leu Gln Leu Gly His Gly Leu Lys Asp
    45                  50                  55

ttt gtc cat aag acg aag ggc caa att aat gac ata ttt caa aaa ctc      302
Phe Val His Lys Thr Lys Gly Gln Ile Asn Asp Ile Phe Gln Lys Leu
60                  65                  70                  75

aac ata ttt gat cag tct ttt tat gat cta tcg ctg caa acc agt gaa      350
Asn Ile Phe Asp Gln Ser Phe Tyr Asp Leu Ser Leu Gln Thr Ser Glu
                80                  85                  90

atc aaa gaa gaa gaa aag gaa ctg aga aga act aca tat aaa cta caa      398
Ile Lys Glu Glu Glu Lys Glu Leu Arg Arg Thr Thr Tyr Lys Leu Gln
            95                  100                 105

gtc aaa aat gaa gag gta aag aat atg tca ctt gaa ctc aac tca aaa      446
Val Lys Asn Glu Glu Val Lys Asn Met Ser Leu Glu Leu Asn Ser Lys
        110                 115                 120

ctt gaa agc ctc cta gaa gaa aaa att cta ctt caa caa aaa gtg aaa      494
Leu Glu Ser Leu Leu Glu Glu Lys Ile Leu Leu Gln Gln Lys Val Lys
    125                 130                 135

tat tta gaa gag caa cta act aac tta att caa aat caa cct gaa act      542
Tyr Leu Glu Glu Gln Leu Thr Asn Leu Ile Gln Asn Gln Pro Glu Thr
140                 145                 150                 155

cca gaa cac cca gaa gta act tca ctt aaa act ttt gta gaa aaa caa      590
Pro Glu His Pro Glu Val Thr Ser Leu Lys Thr Phe Val Glu Lys Gln
                160                 165                 170

gat aat agc atc aaa gac ctt ctc cag acc gtg gaa gac caa tat aaa 638
Asp Asn Ser Ile Lys Asp Leu Leu Gln Thr Val Glu Asp Gln Tyr Lys
            175                 180                 185

caa tta aac caa cag cat agt caa ata aaa gaa ata gaa aat cag ctc 686
Gln Leu Asn Gln Gln His Ser Gln Ile Lys Glu Ile Glu Asn Gln Leu
        190                 195                 200

aga agg act agt att caa gaa ccc aca gaa att tct cta tct tcc aag 734
Arg Arg Thr Ser Ile Gln Glu Pro Thr Glu Ile Ser Leu Ser Ser Lys
    205                 210                 215

cca aga gca cca aga act act ccc ttt ctt cag ttg aat gaa ata aga 782
Pro Arg Ala Pro Arg Thr Thr Pro Phe Leu Gln Leu Asn Glu Ile Arg
220                 225                 230                 235

aat gta aaa cat gat ggc att cct gct gaa tgt acc acc att tat aac 830
Asn Val Lys His Asp Gly Ile Pro Ala Glu Cys Thr Thr Ile Tyr Asn
                240                 245                 250

aga ggt gaa cat aca agt ggc atg tat gcc atc aga ccc agc aac tct 878
Arg Gly Glu His Thr Ser Gly Met Tyr Ala Ile Arg Pro Ser Asn Ser
            255                 260                 265

caa gtt ttt cat gtc tac tgt gat gtt ata tca ggt agt cca tgg aca 926
Gln Val Phe His Val Tyr Cys Asp Val Ile Ser Gly Ser Pro Trp Thr
        270                 275                 280

tta att caa cat cga ata gat gga tca caa aac ttc aat gaa acg tgg 974
Leu Ile Gln His Arg Ile Asp Gly Ser Gln Asn Phe Asn Glu Thr Trp
    285                 290                 295

gag aac tac aaa tat ggt ttt ggg agg ctt gat gga gaa ttt tgg ttg 1022
Glu Asn Tyr Lys Tyr Gly Phe Gly Arg Leu Asp Gly Glu Phe Trp Leu
300                 305                 310                 315

ggc cta gag aag ata tac tcc ata gtg aag caa tct aat tat gtt tta 1070
Gly Leu Glu Lys Ile Tyr Ser Ile Val Lys Gln Ser Asn Tyr Val Leu
                320                 325                 330

cga att gag ttg gaa gac tgg aaa gac aac aaa cat tat att gaa tat 1118
Arg Ile Glu Leu Glu Asp Trp Lys Asp Asn Lys His Tyr Ile Glu Tyr
            335                 340                 345

tct ttt tac ttg gga aat cac gaa acc aac tat acg cta cat cta gtt 1166
Ser Phe Tyr Leu Gly Asn His Glu Thr Asn Tyr Thr Leu His Leu Val
        350                 355                 360

gcg att act ggc aat gtc ccc aat gca atc ccg gaa aac aaa gat ttg 1214
Ala Ile Thr Gly Asn Val Pro Asn Ala Ile Pro Glu Asn Lys Asp Leu
    365                 370                 375

gtg ttt tct act tgg gat cac aaa gca aaa gga cac ttc aac tgt cca 1262
Val Phe Ser Thr Trp Asp His Lys Ala Lys Gly His Phe Asn Cys Pro
380                 385                 390                 395

gag ggt tat tca gga ggc tgg tgg tgg cat gat gag tgt gga gaa aac 1310
Glu Gly Tyr Ser Gly Gly Trp Trp Trp His Asp Glu Cys Gly Glu Asn
                400                 405                 410

aac cta aat ggt aaa tat aac aaa cca aga gca aaa tct aag cca gag 1358
Asn Leu Asn Gly Lys Tyr Asn Lys Pro Arg Ala Lys Ser Lys Pro Glu
            415                 420                 425

agg aga aga gga tta tct tgg aag tct caa aat gga agg tta tac tct     1406
Arg Arg Arg Gly Leu Ser Trp Lys Ser Gln Asn Gly Arg Leu Tyr Ser
        430                 435                 440

ata aaa tca acc aaa atg ttg atc cat cca aca gat tca gaa agc ttt     1454
Ile Lys Ser Thr Lys Met Leu Ile His Pro Thr Asp Ser Glu Ser Phe
    445                 450                 455

gaa tgaactgagg caaatttaaa aggcaataat ttaaacatta acctcattcc          1507
Glu
460

aagttaatgt ggtctaataa tctggtatta aatccttaag agaaagcttg agaaatagat   1567

tttttttatc ttaaagtcac tgtctattta agattaaaca tacaatcaca taaccttaaa   1627

gaataccgtt tacatttctc aatcaaaatt cttataatac tatttgtttt aaattttgtg   1687

atgtgggaat caattttaga tggtcacaa                                     1716

<210>  4

<211>  460

<212>  PRT

<213>  Mus musculus

<400>  4

Met Phe Thr Ile Lys Leu Leu Leu Phe Ile Val Pro Leu Val Ile Ser
1               5                   10                  15

Ser Arg Ile Asp Gln Asp Asn Ser Ser Phe Asp Ser Leu Ser Pro Glu
            20                  25                  30

Pro Lys Ser Arg Phe Ala Met Leu Asp Asp Val Lys Ile Leu Ala Asn
        35                  40                  45

Gly Leu Leu Gln Leu Gly His Gly Leu Lys Asp Phe Val His Lys Thr
    50                  55                  60

Lys Gly Gln Ile Asn Asp Ile Phe Gln Lys Leu Asn Ile Phe Asp Gln
65                  70                  75                  80

Ser Phe Tyr Asp Leu Ser Leu Gln Thr Ser Glu Ile Lys Glu Glu Glu
                85                  90                  95

Lys Glu Leu Arg Arg Thr Thr Tyr Lys Leu Gln Val Lys Asn Glu Glu
            100                 105                 110

Val Lys Asn Met Ser Leu Glu Leu Asn Ser Lys Leu Glu Ser Leu Leu

115                 120                 125

Glu Glu Lys Ile Leu Leu Gln Gln Lys Val Lys Tyr Leu Glu Glu Gln
    130                 135                 140

Leu Thr Asn Leu Ile Gln Asn Gln Pro Glu Thr Pro Glu His Pro Glu
145                 150                 155                 160

Val Thr Ser Leu Lys Thr Phe Val Glu Lys Gln Asp Asn Ser Ile Lys
                165                 170                 175

Asp Leu Leu Gln Thr Val Glu Asp Gln Tyr Lys Gln Leu Asn Gln Gln
            180                 185                 190

His Ser Gln Ile Lys Glu Ile Glu Asn Gln Leu Arg Arg Thr Ser Ile
        195                 200                 205

Gln Glu Pro Thr Glu Ile Ser Leu Ser Ser Lys Pro Arg Ala Pro Arg
    210                 215                 220

Thr Thr Pro Phe Leu Gln Leu Asn Glu Ile Arg Asn Val Lys His Asp
225                 230                 235                 240

Gly Ile Pro Ala Glu Cys Thr Thr Ile Tyr Asn Arg Gly Glu His Thr
                245                 250                 255

Ser Gly Met Tyr Ala Ile Arg Pro Ser Asn Ser Gln Val Phe His Val
            260                 265                 270

Tyr Cys Asp Val Ile Ser Gly Ser Pro Trp Thr Leu Ile Gln His Arg
        275                 280                 285

Ile Asp Gly Ser Gln Asn Phe Asn Glu Thr Trp Glu Asn Tyr Lys Tyr
    290                 295                 300

Gly Phe Gly Arg Leu Asp Gly Glu Phe Trp Leu Gly Leu Glu Lys Ile
305                 310                 315                 320

Tyr Ser Ile Val Lys Gln Ser Asn Tyr Val Leu Arg Ile Glu Leu Glu
                325                 330                 335

Asp Trp Lys Asp Asn Lys His Tyr Ile Glu Tyr Ser Phe Tyr Leu Gly
            340                 345                 350

Asn His Glu Thr Asn Tyr Thr Leu His Leu Val Ala Ile Thr Gly Asn
        355                 360                 365

Val Pro Asn Ala Ile Pro Glu Asn Lys Asp Leu Val Phe Ser Thr Trp
    370                 375                 380

Asp His Lys Ala Lys Gly His Phe Asn Cys Pro Glu Gly Tyr Ser Gly
385                 390                 395                 400

Gly Trp Trp Trp His Asp Glu Cys Gly Glu Asn Asn Leu Asn Gly Lys
                405                 410                 415

Tyr Asn Lys Pro Arg Ala Lys Ser Lys Pro Glu Arg Arg Arg Gly Leu
            420                 425                 430

Ser Trp Lys Ser Gln Asn Gly Arg Leu Tyr Ser Ile Lys Ser Thr Lys
        435                 440                 445

Met Leu Ile His Pro Thr Asp Ser Glu Ser Phe Glu
    450                 455                 460

<210> 5

<211> 23

<212> DNA

<213> Mus musculus

<400> 5
gactgatcaa atatgttgag ctt 23

<210> 6

<211> 21

<212> DNA

<213> Mus musculus

<400> 6
tgcatccaga gtggatccag a 21

<210> 7

<211> 21

<212> DNA

<213> Homo sapiens

<400> 7

tcctctagtt atttcctcca g                                                    21

<210>  8

<211>  20

<212>  DNA

<213>  homo sapiens

<400>  8
tggtttgcca gcgatagatc                                                     20

<210>  9

<211>  14

<212>  PRT

<213>  Artificial Sequence

<220>

<223>  Description of Artificial Sequence: Synthetic
       oligopeptide which is used as an antigen to obtain
       polyclonal antibody

<400>  9

Glu Pro Lys Ser Arg Phe Ala Met Leu Asp Asp Val Lys Cys
1               5                   10

<210>  10

<211>  14

<212>  PRT

<213>  Homo sapiens

<400>  10

Cys Gly Glu Asn Asn Leu Asn Gly Lys Tyr Asn Lys Pro Arg
1               5                   10

<210>  11

<211>  199

<212>  DNA

<213>  Mus musculus

<400> 11
ttgcatccag agtggatcca gacctttcat catttgattc tgcaccttca gagccaaaat 60

caagatttgc tatgttggat gatgtcaaaa ttttagcgaa tggcctcctg cagctgggtc 120

atggacttaa agattttgtc cataagacta agggacaaat taacgacata tttcagaagc 180

tcaacatatt tgatcagtc 199